# EUROPEAN PATENT SPECIFICATION

(11) **EP 2 617 792 B1**
(45) Date of publication and mention of the grant of the patent: **23.12.2020**
(21) Application number: 11825402.8
(22) Date of filing: 09.09.2011
(51) Int. Cl.: C09J 11/00, C09J 193/00, C09J 171/02, A61K 9/00, A61L 24/04, A61K 9/70

(54) **ADHESIVE COMPOSITION COMPRISING TANNIN, POLYETHYLENE GLYCOL, AND WATER, LOWER ALCOHOL OR MIXTURE THEREOF**
KLEBEMASSE AUS TANNIN, POLYETHYLENGLYKOL UND WASSER, NIEDEREM ALKOHOL ODER EINER MISCHUNG DAVON
COMPOSITION ADHÉSIVE COMPRENANT DU TANNIN, DU POLYÉTHYLÈNEGLYCOL, ET DE L'EAU, UN ALCOOL INFÉRIEUR OU UN MÉLANGE DE CEUX-CI

(30) Priority: 14.09.2010 KR 20100090232
(43) Date of publication of application: 24.07.2013
(73) Proprietor: Korea Advanced Institute Of Science And Technology, Daejeon 305-701 (KR); InnoTherapy Inc., Seoul 153-053 (KR)
(72) Inventor: LEE, Haeshin, Daejeon 305-340 (KR); LEE, Moon Sue, Seoul 150-771 (KR); HONG, Seonki, Seoul 158-765 (KR); KIM, Keumyeon, Seoul 138-220 (KR)
(74) Representative: Hoefer & Partner Patentanwälte mbB
(86) International application number: PCT/KR2011/006737
(87) International publication number: WO 2012/036442

(56) References cited:
- WO-A1-99/04764
- JP-A- 2001 510 788
- JP-A- 2002 302 497
- JP-A- 2007 517 776
- YUNAN YU ET AL: "Plasticizing effect of poly(ethylene glycol)s with different molecular weights in poly(lactic acid)/starch blends", JOURNAL OF APPLIED POLYMER SCIENCE, vol. 132, no. 16, 20 April 2015 (2015-04-20), pages n/a-n/a, XP055536164, US ISSN: 0021-8995, DOI: 10.1002/app.41808

## Description

### [Technical Field]

This application claims priority from and the benefit of Korean Patent Application No. 10-2010-0090232, filed on September 14, 2010

The present invention relates to an adhesive composition including tannin, poly(ethylene glycol) and water, lower alcohol or a mixture thereof, and more particularly, to an adhesive composition that is applicable as a bio-adhesive. The adhesive is non-toxic, strong in adhesion force in the presence of moisture and aqueous solution. The applications of the tannin/PEG adhesives include medical adhesives, anti-adhesion agents, hemostatic materials, depots for sustained release of drugs, and anti-fouling surfaces.

### [Background Art]

Adhesives are materials used to attach an object to the surface of another object. According to Internal Organization for Standardization (ISO), 'adhesion' means a state in which two surfaces are integrated by chemical and/or physical force, and 'adhesives' are materials by which integration of two or more objects is allowed. Adhesives have been used widely in various fields, including daily life and industrial work due to their handiness. Such adhesives may be classified into inorganic adhesives and organic adhesives depending on the particular types of their main ingredients. Organic adhesives may be further classified into synthetic organic adhesives and natural organic adhesives. Particularly, synthetic organic adhesives may be further classified into resin-based adhesives including thermosetting resins or thermoplastic resins, rubber-based adhesives, and mixed adhesives including phenolic adhesives and epoxy adhesives. However, in the case of synthetic organic adhesives, emission of harmful substances has become a serious problem recently. Such problems are caused by the use of volatile organic solvents and evaporation of unreacted monomers. More particularly, in the case of adhesives for use in interior materials for buildings, strict regulations have been applied to emission of harmful substances, such as formaldehyde. Under these circumstances, glue-, soy bean protein- and tannin-based adhesives have been developed.

The term 'repeatable adhesive' may be regarded as a kind of adhesive, and means a material that is adhered sufficiently to an adherent merely by pressure (finger pressure) applied to a portion of adhesive, has lower cohesive force and more rapid stress relaxation as compared to an adhesive, and shows visco-elastic behaviors permitting easy deformation against external force. Raw materials used for such repeatable adhesives include natural rubber, synthetic rubber and thermoplastic resins, such as acrylic or silicone resins, and are characterized by their elasticity. Thus, unlike adhesives, repeatable adhesives are capable of maintaining their adhesion strength even when used repeatedly many times, and adhere due to their property of so-called stickiness.

Tannin adhesives include, as a main ingredient, tannin that is contained in the bark of trees, such as mimosa wattle, quebracho and radiata pine in a large amount, and are used particularly for the adhesion of wood materials. As a curing agent for such tannin adhesives, a formaldehyde-based curing agent, such as formalin, paraformaldehyde or hexamethylene tetramine (hexamine), is frequently used. Since a formaldehyde-based curing agent induces condensation by forming a methylene bridge between tannin and tannin or between tannin and wood, tannin adhesives have been spotlighted as eco-friendly adhesives emitting no formaldehyde. However, since tannin adhesives are thermosetting, their curing occurs at a temperature of 100°C or higher, and they have higher adhesion strength as the temperature increases. In addition, because tannin adhesives are water soluble, it is difficult for them to maintain their adhesion strength in an aqueous environment.

Although tannin adhesives have been spotlighted as non-toxic natural adhesives, their application is limited because they have water solubility and they require a thermosetting curing agent. Therefore, there has been a continuous need for an adhesive that is not harmful to the human body, has little water solubility to be used in an aqueous environment, and requires no heating upon curing. WO99/04764 A1 discloses a liquid composition comprising a polymer and tannic acid or tannin which can form an adhesive solid composition capable of controlled release of a pharmaceutical. The composition optionally includes a plasticizer, e.g. polyethylene glycol. The composition can be made as follows: the polymer is dissolved in a solvent, tannic acid or tannin is added in a solvent, a precipitate is allowed to form, the precipitate is dried to form the solid composition. The solvent is water, ethanol, mixtures of water and ethanol, isopropanol, mixtures of water and isopropanol, n-propanol, and mixtures of water and n-propanol.

### [Disclosure]

### [Technical Problem]

We have conducted many studies to develop an adhesive that is non-toxic, is not harmful to the human body, has moisture resistance and requires no heating upon curing. We have found that an adhesive including tannin in combination with poly(ethylene glycol) has little water solubility, and allows adhesion in the absence of heating. The present invention is based on these findings.

The present invention is directed to providing an adhesive composition as defined in the claims comprising tannin, poly(ethylene glycol), and water, lower alcohol or a mixture thereof.

The adhesive composition is for use in a method of adhering tissue or organs in vivo.

The present invention is also directed to providing a medical adhesive comprising the adhesive composition.

The present invention is also directed to a adhesive depot for sustained release of drug (controlled released drug delivery adhesives) comprising the adhesive composition.

The present invention is also directed to a anti-adhesion agent comprising the adhesive composition.

The present invention is also directed to a surface adsorption barrier comprising the adhesive composition.

The present invention is also directed to a medical hemostatic comprising the adhesive composition.

The present invention is also directed to a method for preparing an adhesive composition, comprising: mixing tannin, poly(ethylene glycol), and water, lower alcohol or a mixture thereof; and acquiring the resultant mixture as an adhesive composition.

The present invention is also directed to a method for preparing an adhesive composition, comprising: dissolving poly(ethylene glycol) into water, lower alcohol or a mixed solvent thereof; dissolving tannin into water, lower alcohol or a mixed solvent thereof; mixing the solutions obtained from the above operations with each other to form an adhesive composition; and acquiring the resultant adhesive composition.

The present invention is also directed to an adhesive composition obtained by the above-described methods.

The present description also discloses a method for adhering two or more separate surfaces comprising the steps of: (a) applying a adhesive composition comprising tannin; poly(ethylene glycol); and water, lower alcohol or a mixture thereof onto the surfaces; and (b) contacting the surfaces each other.

The present description also discloses to use of a composition comprising tannin, poly(ethylene glycol), and water, lower alcohol or a mixture thereof for the manufacture of an adhesive agent.

The present description also discloses to use of a composition comprising tannin, poly(ethylene glycol), and water, lower alcohol or a mixture thereof for the manufacture of an medical adhesive.

The present description also discloses to use of a composition comprising tannin, poly(ethylene glycol), and water, lower alcohol or a mixture thereof for the manufacture of a adhesive drug formulation for sustained release.

The present description also discloses to use of a composition comprising tannin, poly(ethylene glycol), and water, lower alcohol or a mixture thereof for the manufacture of a anti-adhesion agent.

The present description also discloses to use of a composition comprising tannin, poly(ethylene glycol), and water, lower alcohol or a mixture thereof for the manufacture of a surface adsorption barrier.

The present description also discloses to use of a composition comprising tannin, poly(ethylene glycol), and water, lower alcohol or a mixture thereof for the manufacture of a medical hemostatic.

### [Technical Solution]

In one general aspect, the present invention provides an adhesive composition as defined in the claims comprising tannin, poly(ethylene glycol), and water, lower alcohol or a mixture thereof.

The adhesive composition is for use in a method of adhering tissue or organs in vivo.

In another general aspect, the present invention provides a medical adhesive comprising the adhesive composition.

In still another general aspect, the present invention provides a adhesive depot for sustained release of drug (controlled released drug delivery adhesives) comprising the adhesive composition.

In still another general aspect, the present invention provides a anti-adhesion agent comprising the adhesive composition.

In still another general aspect, the present invention provides a surface adsorption barrier comprising the adhesive composition.

In still another general aspect, the present invention provides a medical hemostatic comprising the adhesive composition.

In still another general aspect, the present invention provides a method for preparing an adhesive composition, comprising: mixing tannin, poly(ethylene glycol), and water, lower alcohol or a mixture thereof; and acquiring the resultant mixture as an adhesive composition.

In still another general aspect, the present invention provides a method for preparing an adhesive composition, including: dissolving poly(ethylene glycol) into water, lower alcohol or a mixed solvent thereof; dissolving tannin into water, lower alcohol or a mixed solvent thereof; mixing the solutions obtained from the above operations with each other to form an adhesive composition; and acquiring the resultant adhesive composition.

In yet another general aspect, the present invention provides an adhesive composition obtained by the above-described methods.

The description also discloses a method for adhering two or more separate surfaces comprising the steps of: (a) applying a adhesive composition comprising tannin; poly(ethylene glycol); and water, lower alcohol or a mixture thereof onto the surfaces; and (b) contacting the surfaces each other.

The description also discloses use of a composition comprising tannin, poly(ethylene glycol), and water, lower alcohol or a mixture thereof for the manufacture of an adhesive agent.

The description also discloses use of a composition comprising tannin, poly(ethylene glycol), and water, lower alcohol or a mixture thereof for the manufacture of an medical adhesive.

The description also discloses use of a composition comprising tannin, poly(ethylene glycol), and water, lower alcohol or a mixture thereof for the manufacture of a adhesive drug formulation for sustained release.

The description also discloses use of a composition comprising tannin, poly(ethylene glycol), and water, lower alcohol or a mixture thereof for the manufacture of a anti-adhesion agent.

The description also discloses use of a composition comprising tannin, poly(ethylene glycol), and water, lower alcohol or a mixture thereof for the manufacture of a surface adsorption barrier.

The description also discloses use of a composition comprising tannin, poly(ethylene glycol), and water, lower alcohol or a mixture thereof for the manufacture of a medical hemostatic.

### [Advantageous Effects]

The adhesive composition disclosed herein, including tannin, poly(ethylene glycol) and water, has little toxicity, allows adhesion even in the absence of a thermosetting curing agent, and is hardly soluble in water to show moisture resistance, unlike known tannin adhesives. Therefore, the adhesive composition provides a tannin-based adhesive applicable as a medical adhesive, a adhesive depot for sustained release of drug (controlled released drug delivery adhesives), a anti-adhesion agent, a cell/protein adsorption barrier and a medical hemostatic.

### [Description of Drawings]

The above and other objects, features and advantages of the present invention will become apparent from the following description of certain exemplary embodiments given in conjunction with the accompanying drawings, in which:
Fig. 1 is a photograph that shows an adhesive obtained by mixing tannin, poly(ethylene glycol) (PEG) and water according to an embodiment of the present invention;

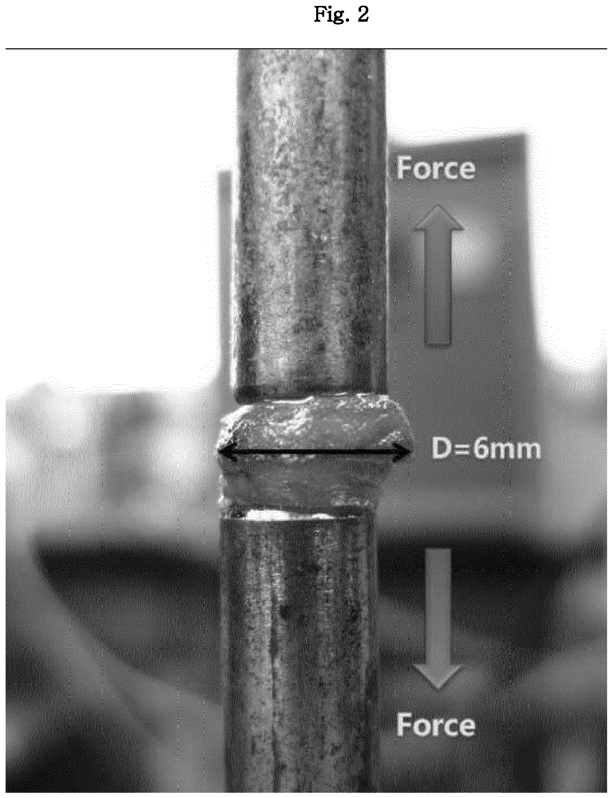

Fig. 2 is a photograph that shows a test method for determining the adhesion strength of the adhesive according to the present invention;

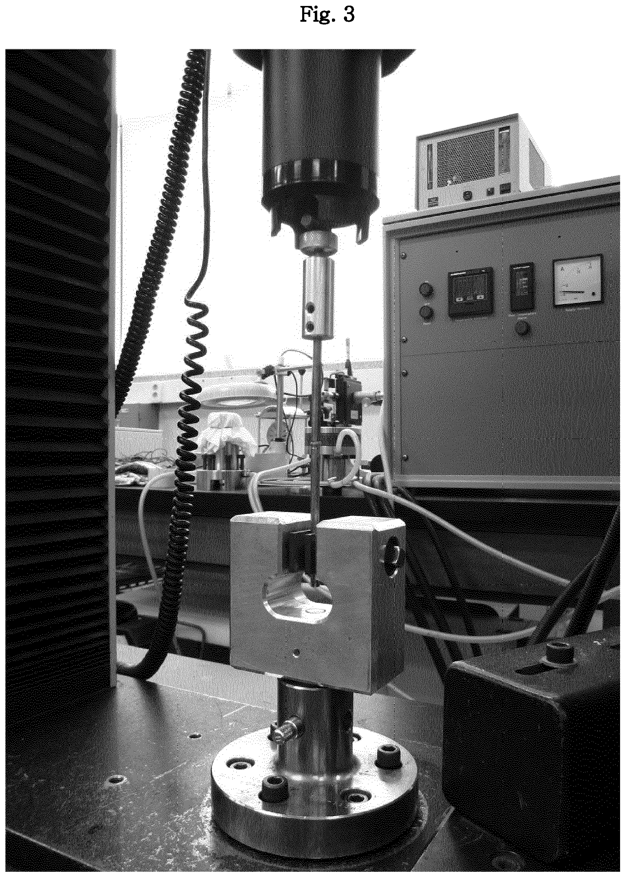

Fig. 3 is a photograph that shows an instrument for determining the adhesion strength in the test of Fig.2;
Fig. 4 is a chart that shows the test results of the adhesion strength of an adhesive including tannin, water and linear PEG;
Fig. 5 is a chart that shows the test results of the adhesion strength of an adhesive including tannin, multi-branched PEG, and water or a lower alcohol (methanol or ethanol), as compared to a commercially available bond (Doae Ji Pyo Bond available from Daeheung Chemical Co.) and an adhesive for stationery;
Fig. 6 is a graph showing the adhesion strength of the adhesives, 4-arm-PEG-OH (10 kDa) and 4-arm-PEG-AM (10 kDa), as a function of the number of repeated adhesion tests;
Fig. 7 is a graph showing the test results of moisture resistance expressed by the adhesion strength after applying distilled water to the adhesive, 4-arm-PEG-OH (10 kDa);
Fig. 8 is a graph showing the test results of moisture resistance expressed by the adhesion strength after applying distilled water to the adhesive, 4-arm-PEG-AM (10 kDa);
Fig. 9 is a graph that shows the biodegradability of the adhesive according to the present invention by determining tannin release as a function of pH;
Fig. 10 is a graph that shows the drug delivery capability of the adhesive depot for sustained release of drug (controlled released drug delivery adhesives) using the adhesive according to the present invention by determining the protein release rate as a function of the concentration of protein delivered by the drug delivery adhesive;
Fig. 11 is a chart that shows the variations in adhesion strength when 4-arm-PEG-OH (10 kDa) is mixed with fluorescein isothiocyanate labeled bovine serum albumin (FITC-BSA);
Fig. 12 is a graph that shows the variations in adhesion strength as a function of number of repeated adhesion tests, when 4-arm-PEG-OH (10 kDa) is mixed with FITC-BSA;
Fig. 13 is a chart that shows the variations in adhesion strength when 4-arm-PEG-AM (10 kDa) is mixed with FITC-BSA;
Fig. 14 is a graph that shows the variations in adhesion strength as a function of number of repeated adhesion tests, when 4-arm-PEG-OH (10 kDa) is mixed with FITC-BSA;
Fig. 15 is a fluorescence microscopic image that shows the results of the number of cells remaining on a non-surface modified gold surface and on a gold surface modified with the adhesive according to the present invention; and
Fig. 16 is a chart that shows the results of the number of cells remaining on a gold surface modified with an adhesive using 4-arm-PEG-AM (10 kDa) or with an adhesive using 4-arm-PEG-OH (10 kDa) after storing the adhesives (1) at room temperature, (2) at 60°C and (3) at 60°C for 1 hour.
Fig. 17 is a photograph showing how the adhesive obtained by using PEG (4.6 kDa) is decomposed with time.

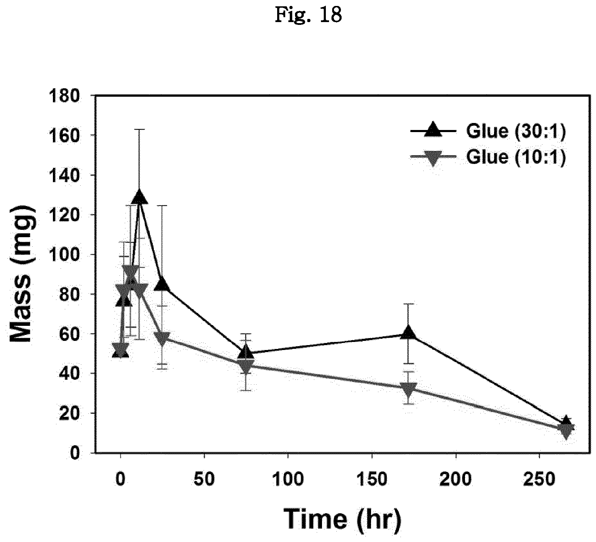

Fig. 18 is a graph showing the results of the test of decomposition of the adhesive obtained by using PEG (4.6 kDa) as expressed by measuring the weight thereof.
Fig. 19 is a graph showing the effects of a negative control (non-treated), a positive control (Fibrin glue, Beriplast P combi-set) and the PEG adhesive (4.6k, pyrogallol:hydroxy group = 30:1) disclosed herein upon hemostasis, as determined by measuring the amount of blood for 2 minutes at an interval of 30 seconds.
Fig. 20 is a chart showing the effects of a negative control, a positive control (Fibrin glue, Beriplast P combi-set) and the PEG adhesive (4.6k, pyrogallol:hydroxy group = 30:1) disclosed herein upon hemostasis, as determined by measuring the total amount of blood flowing out for 2 minutes.

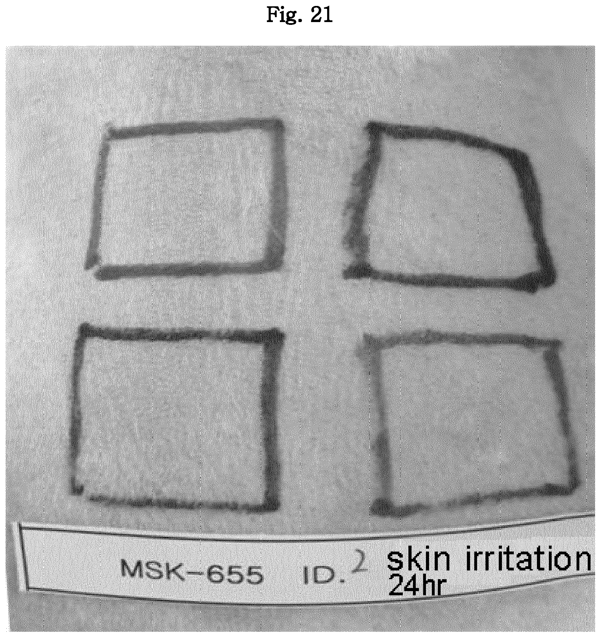

Fig. 21 is a photograph taken at the skin site of a male rabbit, 24 hours after applying the PEG adhesive (4.6k, pyrogallol:hydroxy group = 10:1) disclosed herein thereto according to ISO 10993-10:2002/Amd.1:2006(E), maximization test for delayed hypersensitivity.

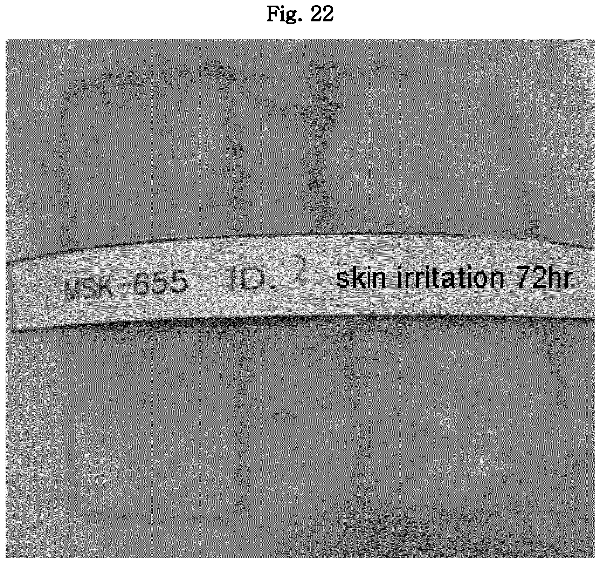

Fig. 22 is a photograph taken at the skin site of a male rabbit, 72 hours after applying the PEG adhesive (4.6k, pyrogallol:hydroxy group = 10:1) disclosed herein thereto according to ISO 10993-10:2002/Amd.1:2006(E), maximization test for delayed hypersensitivity

### [Best Mode]

Hereinafter, the embodiments of the present invention will be described in detail with reference to accompanying drawings.

In one aspect, there is provided an adhesive composition as defined in the claims including tannin, poly(ethylene glycol) (PEG), and water, lower alcohol or a mixture thereof.

The adhesive composition is for use in a method of adhering tissue or organs in vivo.

Tannins are phenolic compounds extracted mainly from the core or bark of trees. Aqueous tannin solution is a generic term of compounds having high astringent property and an astringent taste, and includes polymeric materials obtained by polymerization of many kinds of polyphenols and having a complicated structure. Such aqueous tannin solution is colorless in nature but is oxidized easily by the action of polyphenol oxidase to show a brown color.

Tannins may be further classified into hydrolysable tannins and condensed tannins, both of which are included in the present invention. Hydrolysable tannins are extracted from a chestnut tree, myrobolans, dividivi, etc., are single molecular phenolic compounds, such as pyrogallol, gallic acid or ellagic acid, and have been used as a substitute for a part of phenol during the preparation of phenolic resins. It has been known that such hydrolysable tannins are not suitable for adhesives in terms of economics and chemistry as compared to condensed tannins, because they have low reactivity to formaldehyde and low availability. However, hydrolysable tannins may be useful in the adhesive composition disclosed herein. Condensed tannins form water-insoluble plobaphene via polymerization, are frequently used as ingredients of conventional tannin-based adhesives, and occupy a significant portion of the bark of some trees.

Tannins used in the adhesive composition disclosed herein may include, but are not limited to: pyrogallol, gallic acid or ellagic acid of the following Chemical Formula 1; gallic acid of the following Chemical Formula 2; ellagic acid of the following Chemical Formula 3; catechin of the following Chemical Formula 4; polymers thereof; and other types of tannins described in the known publication, Cesar G. Fraga, Plant Phenolics and Human Health Biochemistry, Nutrition, and Pharmacology, 2010, Wiley, ISBN: 978-0-470-28721-7. Such tannins may have a molecular weight (MW) of 500-10,000 but are not limited thereto.

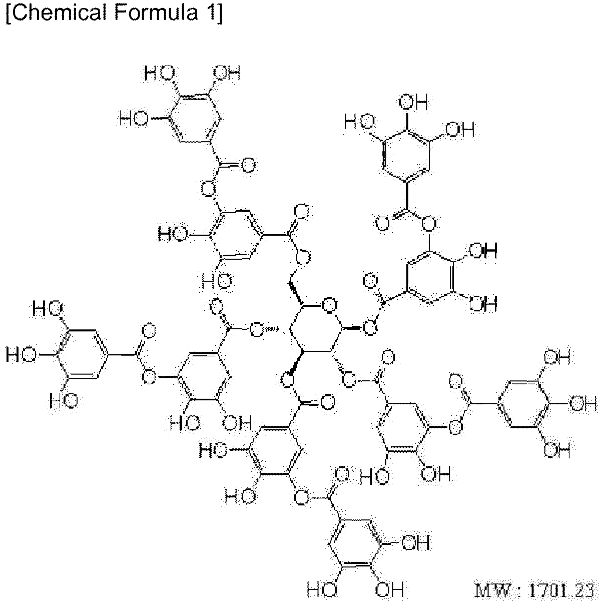

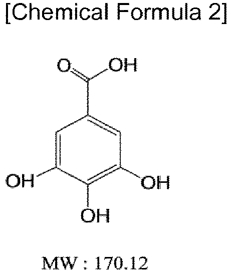

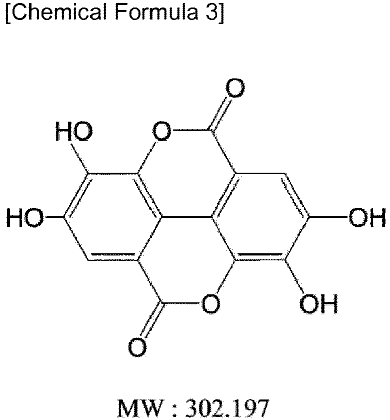

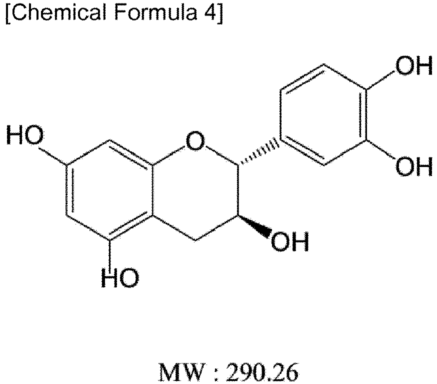

Poly(ethylene glycol) (PEG) is a non-toxic polymeric material having high solubility, and thus is widely used in various industrial fields, including food, cosmetics and ceramics. PEG has a different melting point and melting heat depending on molecular weight, and thus PEG having an adequate molecular weight is selected depending on the intended use. However, PEG having a molecular weight of 100,000 daltons or less is used.

PEG used herein includes linear or multi-branched one, and may have a functional group. Non-limiting examples of the functional group include: hydroxyl (-OH), amine (-NH₂), succinimidyl succinate, succinimidyl glutarate, succinic acid, thiol (-SH), acrylate, epoxide, maleimide, nitrophenyl carbonate, orthopyridyl disulfide, tosylate, azide, or phosphate. Other examples of the functional group include oligoamine ([-CH₂-CH₂-NH-]ₙ), catechol or catecholamine. In other words, PEG used herein may be linear and particular examples thereof include mPEG-functional group, functional group-PEG-functional group, multi-branched 4-arm-PEG-functional group, 6-arm-PEG-functional group. PEG used herein may be a compound represented by any one of the following Chemical Formulae 5 to 30, but is not limited thereto. In the following Chemical Formulae, n may be an integer ranging from 20 to 2500 but is not limited thereto.

More particularly, Chemical Formulae 5 to 15 represent linear PEGs in the form of mPEG-functional group as follows: Chemical Formula 5 has hydroxyl (-OH) as a functional group; Chemical Formula 6 has amine (-NH₂) as a functional group; Chemical Formula 7 has succinimidyl succinate as a functional group; Chemical Formula 8 has succinic acid as a functional group; Chemical Formula 9 has thiol (-SH) as a functional group; Chemical Formula 10 has acrylate as a functional group; Chemical Formula 11 has epoxide as a functional group; Chemical Formula 12 has maleimide as a functional group; Chemical Formula 13 has nitrophenyl carbonate as a functional group; Chemical Formula 14 has orthopyridyl disulfide as a functional group; and Chemical Formula 15 has tosylate as a functional group.

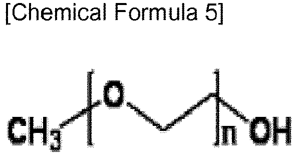

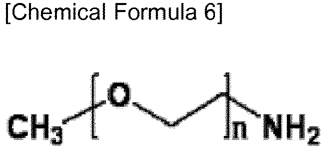

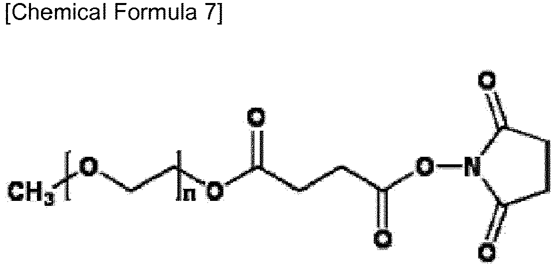

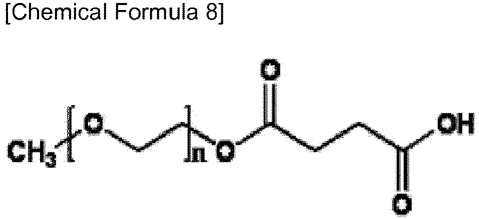

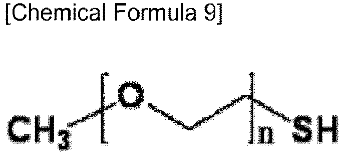

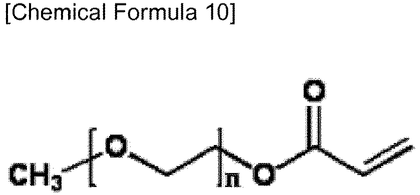

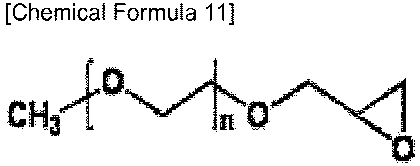

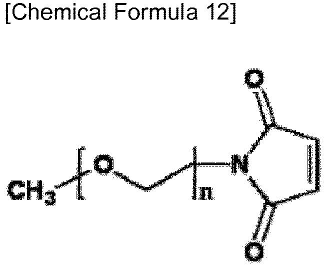

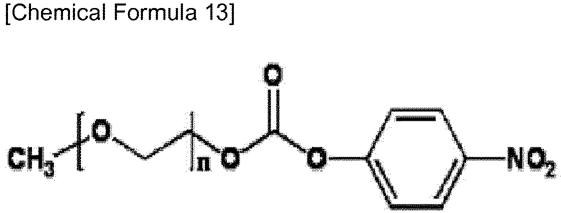

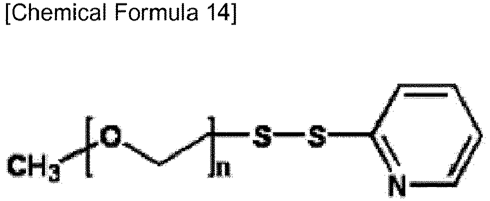

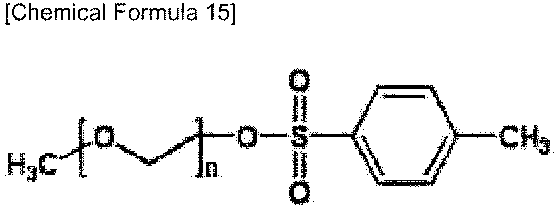

In addition, Chemical Formulae 16 to 26 represent linear PEGs in the form of functional group-mPEG-functional group as follows: Chemical Formula 16 has hydroxyl (-OH) as a functional group; Chemical Formula 17 has amine (-NH₂) as a functional group; Chemical Formula 18 has succinimidyl succinate as a functional group; Chemical Formula 19 has succinic acid as a functional group; Chemical Formula 20 has thiol (-SH) as a functional group; Chemical Formula 21 has acrylate as a functional group; Chemical Formula 22 has epoxide as a functional group; Chemical Formula 23 has maleimide as a functional group; Chemical Formula 24 has nitrophenyl carbonate as a functional group; Chemical Formula 25 has orthopyridyl disulfide as a functional group; and Chemical Formula 26 has tosylate as a functional group.

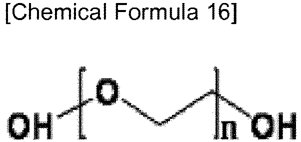

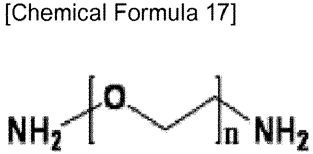

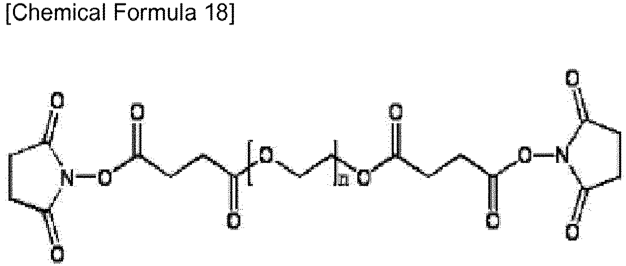

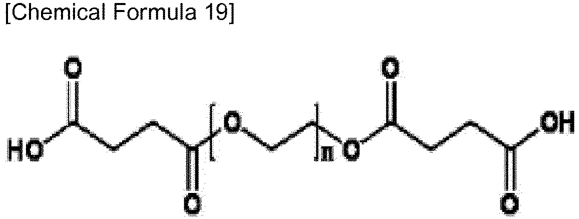

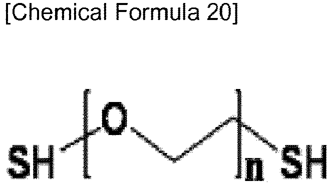

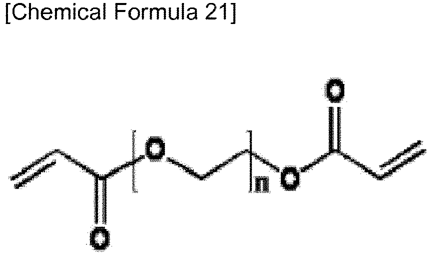

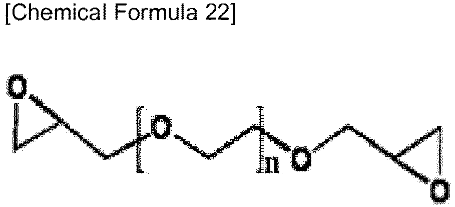

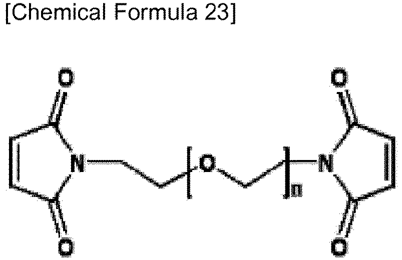

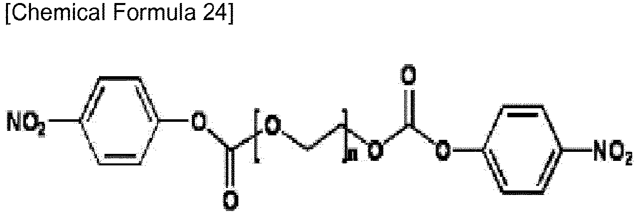

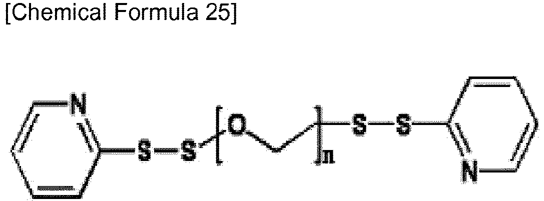

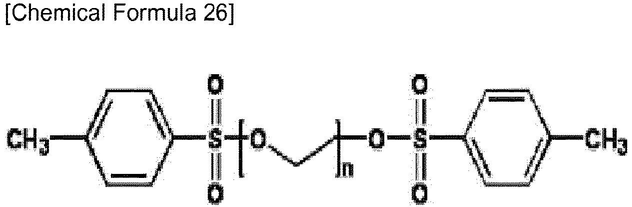

The following Chemical Formula 27 represents multi-branched PEG in the form of 4-arm-PEG having amine as a functional group.

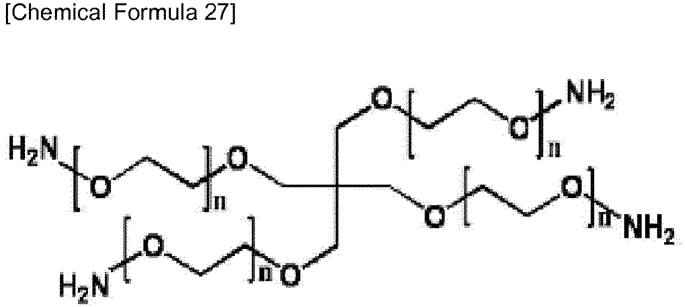

The following Chemical Formula 28 represents 4-arm-PEG that may be substituted with a functional group (hereinafter, shown in sphere in chemical formula) selected from hydroxyl (-OH), succinimidyl succinate, succinic acid, thiol (-SH), acrylate, epoxide, maleimide, nitrophenyl carbonate, orthopyridyl disulfide and tosylate.

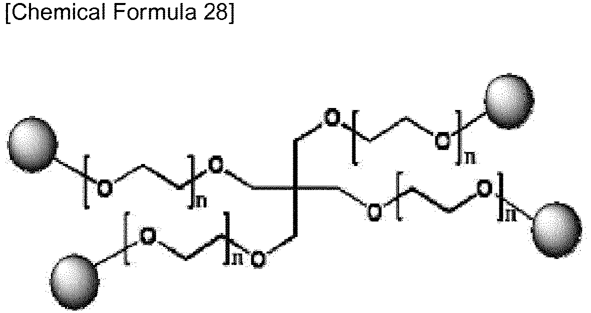

The following Chemical Formula 29 represents 6-arm-PEG having amine as a functional group.

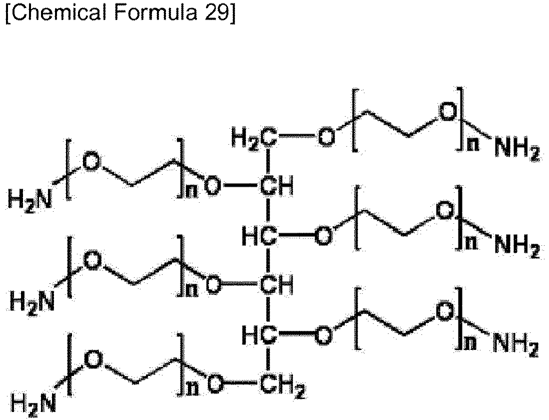

The following Chemical Formula 30 represents 6-arm-PEG that may be substituted with a functional group selected from hydroxyl (-OH), succinimidyl succinate, succinic acid, thiol (-SH), acrylate, epoxide, maleimide, nitrophenyl carbonate, orthopyridyl disulfide and tosylate.

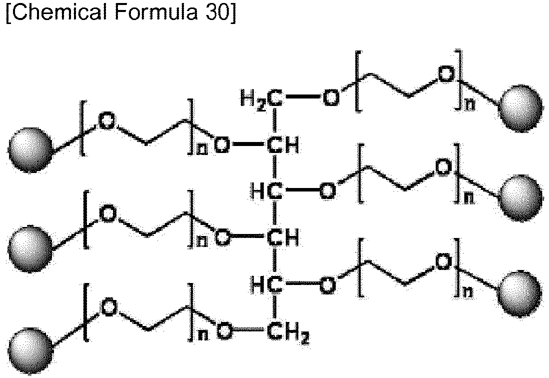

The above-listed PEGs that are used in the present invention have a molecular weight of 1,000-100,000, more particularly 2,000-30,000.

The adhesive composition disclosed herein further includes water, water miscible solvent such as lower alcohol, or a mixture thereof. The term 'lower alcohol' means alcohol having a small number of carbon atoms (1-6 carbon atoms). Such lower alcohols are soluble in water but are neutral substances that are not ionized electrochemically and generate no ions. The OH groups in such lower alcohols are different in nature from the OH groups of bases, and thus show no alkalinity. Particular non-limiting examples of lower alcohol include methanol, ethanol, n-propanol, isopropanol, butanol, amyl alcohol, n-amyl alcohol, n-hexyl alcohol, etc. Particularly, ethanol may be used as lower alcohol for the adhesive composition disclosed herein.

The adhesive composition may further include additives capable of improving the physical properties, and such additives may be selected as desired by those skilled in the art.

In a general point of view, 'adhesion' means a state that includes solidification at an initial time, followed by conversion into a non-elastic state, and has difficulties in repeated attachment. However, as used herein, 'adhesion' has a meaning including 'repeatable adhesion', which means a state that allows easy attachment under pressure while maintaining visco-elastic properties and permits repeated attachment many times after detachment. The adhesive composition disclosed herein has properties as a repeatable adhesive.

In a non-limiting embodiment, the adhesive composition includes 30-80 wt% of tannin, 20-70 wt% of PEG and 5-30 wt% of water, lower alcohol or a mixture thereof. In a particular embodiment, the adhesive composition may include 40-70 wt% of tannin, 30-60 wt% of PEG and 10-25 wt% of water, lower alcohol or a mixture thereof. More particularly, the adhesive composition may include 50-60 wt% of tannin, 40-50 wt% of PEG and 15-20 wt% of water, lower alcohol or a mixture thereof.

The adhesive composition is non-toxic. Therefore, the adhesive composition may be applied as a medical adhesive and may be in direct contact with skin (See Example 11 and 12). Even if the adhesive composition is applied to an in vivo environment and flows into the body fluid and blood to be incorporated into the in vivo environment, it shows no toxicity and harm.

In addition, the adhesive disclosed herein has water resistance so that it may be applied to an aqueous environment. The term 'water resistance' means property by which the adhesive maintains its adhesion strength in an environment including water, such as moisture. It is required for the adhesive to be hardly soluble in aqueous solution or high-humidity environment and to maintain its adhesion strength stably even in an environment including fluid flow in order to show water resistance. The adhesive disclosed herein is hardly soluble in water, and thus may be used as an adhesive having water resistance.

In another aspect, there is provided a medical adhesive comprising the adhesive composition disclosed herein. Also, there is provided a medical hemostatic including adhesive composition disclosed herein.

The term 'medical adhesive' means, in a broad point of view, an adhesive applicable to a wide range of fields, including packaging of medical instruments and surgical sticking, adhesion and hemostasis, and having biocompatibility so that it may be used directly on the skin. In general, a medical adhesive means an adhesive having no toxicity and harm when incorporated into an in vivo environment so as to be used in an in vivo environment. It is required for an adhesive to have biodegradability, water resistance, sterilizing property, non-toxicity and hemostatic effect in addition to biocompatibility and to cause no interruption upon treating the living body so that it may be used as a medical adhesive. The adhesive disclosed herein is applicable to the use as a medical adhesive, including the use as a sticker. More particularly, the adhesive may be used in an in vivo environment as a hemostatic adhesive for surgery and a medical sealant used after large intestine laparotomy.

In an embodiment, the adhesive disclosed herein is subjected to a test for measuring adhesion strength to determine its effect. After the test, it is shown that the adhesive maintains higher adhesion strength than a commercially available adhesive for stationery and lower adhesion strength than a commercially available bond (Doae Ji Pyo Bond available from Daeheung Chemical Co.) (see Example 2).

In another embodiment, the adhesive is tested for its adhesion strength after repeating adhesion under force with constant strength to determine whether it maintains adhesion strength during repeated use or not. As a result, it is shown that the adhesive maintains adhesion strength after repeating adhesion. This suggests that the adhesive composition disclosed herein may also be used as a repeatable adhesive (see Example 3).

In still another embodiment, the adhesive is tested for water resistance by measuring the initial adhesion strength and then determining whether the adhesive maintains its adhesion strength after applying distilled water thereto or not. As a result, it is shown that the adhesive disclosed herein maintains adhesion strength to a predetermined degree of strength or higher even in an environment including moisture. This suggests that the adhesive may be used as a medical adhesive in an in vivo environment as well as in vitro environment (See Example 4).

In one example of the present invention, the degradability of the inventive adhesive composition was measured when the inventive adhesive composition was placed at a temperature similar to the human body temperature, in order to determine the degradable characteristics of the inventive adhesive. As a result, it could be seen that the inventive adhesives are decomposed naturally as time goes. Therefore, it is believed that although the inventive adhesive is water-resistant, it may be decomposed after retained in the living body for a long time (see Example 9).

In one example of the present invention, in order to determine that the inventive adhesive could act as surgical adhesive hemostatic (or medical hemostatic), the effect of the inventive adhesive composition on hemostatis was examined by referring the method disclosed in a published paper (Y. Murakami et al., Colloids and Surfaces B: Biointerfaces 65 (2008) 186-189). As a result the inventive adhesive has superior effect than commercialized hemostatics, Fibrin glue (see Example 10).

In still another aspect, there is provided a adhesive depot for sustained release of drug (controlled released drug delivery adhesives) including the adhesive composition disclosed herein.

The term 'adhesive depot for sustained release of drug' means a formulation designed to release a therapeutically required amount of drug over a longer period of time than a general formulation. Although different terms are used in different countries, such formulations are generally referred to as a sustained release formulation. The sustained release formulation causes a rapid increase in drug concentration in blood to reach a therapeutically required concentration and maintains a constant effective concentration in blood for a desired period of time. Unlike a general formulation, the adhesive depot for sustained release of drug of the present invention requires a lower administration frequency and is designed to maintain biological reactions constantly and to reduce side effects. The adhesive composition disclosed herein may be used to prepare a adhesive depot for sustained release of drug, and may include hardly soluble drugs, therapeutic peptides, proteins and antibodies that may be delivered by the adhesive depot for sustained release of drug of the present invention. Non-limiting examples of the hardly soluble drugs may include paclitaxel or doxorubicin having a small molecular weight, and those of the therapeutic peptides include somatostatin, calcitonin, vasopressin, platelet aggregate inhibitors, gornadotropin-releasing hormone, etc. Non-limiting examples of the proteins and antibodies may include interleukin family, erythropoietin (EPO), granulocyte colony-stimulating factor (G-CSF), human growth hormone, etc.

In an embodiment, the adhesive disclosed herein is tested for its quality as a drug delivery system by investigating protein release after introducing the protein into adhesives using 4-arm-PEG-AM (10kDa) and 4-arm-PEG-OH (10 kDa). As a result, it is shown that the protein is released gradually over a long period of time. This suggests that the adhesive composition disclosed herein may be used as a adhesive depot for sustained release of drug (see Examples 5 and 6).

In still another aspect, there is provided a anti-adhesion agent including the adhesive composition disclosed herein.

The term 'anti-adhesion agent' (adhesion barrier) means a material that remains at a site where adhesion may occur as a physical barrier to prevent adhesion between the adjacent tissues. Therefore, such anti-adhesion agents have to be de decomposed after a predetermined time, should not be absorbed into the living body and should not remain in the living body as foreign materials. The adhesive composition disclosed herein may be incorporated into anti-adhesion agent preparations capable of preventing adhesion that may occur after surgery.

In an embodiment, a test is carried out to determine whether cell attachment occurs or not on a gold surface modified with the adhesive composition disclosed herein in order to confirm the function of the adhesive composition as an anti-adhesion agent. As a result, it is shown that the gold surface modified with the adhesive disclosed herein has little cells remaining thereon as compared to a non-surface modified gold surface. This suggests that the adhesive composition disclosed herein may serve as an anti-adhesion agent (see Example 8).

In still another aspect, there is provided a surface adsorption barrier including the adhesive composition disclosed herein.

In still another aspect, there is provided a method for preparing an adhesive composition, including: mixing tannin, poly(ethylene glycol), and water, lower alcohol or a mixture thereof; and acquiring the resultant mixture as an adhesive composition. Hereinafter, the method will be explained in more detail.

In the mixing operation, tannin, poly(ethylene glycol), and water, lower alcohol or a mixture thereof are mixed to provide the adhesive composition disclosed herein. Upon the mixing, the above materials may be mixed in any desired order and the mixing order is not limited to the above-described order. When tannin is decomposed, it may be converted into pyrogallol. According to an embodiment, molar ratio of pyrogallol to the functional groups of poly(ethylene glycol) may be 20-30:1. More particularly, pyrogallol and poly(ethylene glycol) are weighed and mixed in such a manner that the molar ratio is 25:1 (see Examples 1 and 2).

Then, the mixture obtained from the mixing operation is acquired as an adhesive composition.

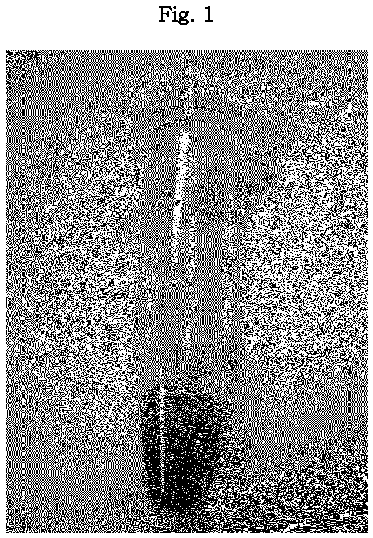

The mixture obtained from the mixing operation causes no additional reaction and provides an adhesive composition as it is without a need for additional operations. When the mixture is allowed to stand for 8-12 minutes after mixing, it is separated into an upper layer and a lower layer, wherein the lower layer contains a brown-colored material, which corresponds to the adhesive composition disclosed herein (see Fig. 1).

In still another aspect, there is provided a method for preparing an adhesive composition, including: dissolving poly(ethylene glycol) into water, lower alcohol or a mixed solvent thereof; dissolving tannin into water, lower alcohol or a mixed solvent thereof; mixing the solutions obtained from the above operations (two dissolving steps) with each other to form an adhesive composition; and acquiring the resultant adhesive composition. Hereinafter, the method will be explained in more detail.

First, poly(ethylene glycol) is dissolved into water, lower alcohol or a mixed solvent thereof. Next, tannin is dissolved into water, lower alcohol or a mixed solvent thereof.

The method for preparing the adhesive composition disclosed herein includes dissolving poly(ethylene glycol) into water, lower alcohol or a mixed solvent thereof, and dissolving tannin into water, lower alcohol or a mixed solvent thereof. The ingredients used for the adhesive composition disclosed herein, i.e., tannin and poly(ethylene glycol), are separately dissolved into water, lower alcohol or a mixed solvent thereof so that they are provided in easily miscible states. Dissolving tannin and dissolving poly(ethylene glycol) are carried out independently from each other. In addition, when dissolving the ingredients, buffering solutions may be used to adjust pH (see Example 1).

Then, the solutions obtained from the above dissolving operations are mixed with each other to form an adhesive composition.

In the mixing operation, the tannin solution and the poly(ethylene glycol) solution are mixed with each other to form an adhesive composition. The mixing order is not limited to the above-described order and the mixing ratio is the same as described above.

Finally, the mixture obtained from the mixing operation is acquired as an adhesive composition.

The mixture obtained from the mixing operation causes no additional reaction and provides an adhesive composition as it is without a need for additional operations. As mentioned earlier, the brown-colored material as shown in Fig. 1 corresponds to the adhesive composition disclosed herein.

In yet another aspect, there is provided an adhesive composition obtained by the above-described methods. The adhesive composition disclosed herein may be obtained by the above-described methods but is not limited thereto.

Meanwhile, the present description discloses a method for adhering two or more separate surfaces comprising the steps of:
(a) applying a adhesive composition comprising tannin; poly(ethylene glycol); and water, lower alcohol or a mixture thereof on the surfaces; and
(b) contacting the surfaces each other.

The description also discloses use of a composition comprising tannin, poly(ethylene glycol), and water, lower alcohol or a mixture thereof for the manufacture of an adhesive agent.

The description also discloses use of a composition comprising tannin, poly(ethylene glycol), and water, lower alcohol or a mixture thereof for the manufacture of an medical adhesive.

The description also discloses use of a composition comprising tannin, poly(ethylene glycol), and water, lower alcohol or a mixture thereof for the manufacture of an adhesive drug formulation for sustained release.

The description also discloses a method for adhering tissues or organs of animal comprising the steps of :
(a) applying the adhesive composition of the present invention on one or both contact site of the tissues or organs of animal; and
(b) contacting the contact site of the tissues or organs of animal each other.

By the contact of the surface or the contact site of the tissues or organs of animal, the two (or more) surfaces or the contact sites were adhered to each other.

The drug may include, but not limited thereto, chemicals, proteins, nucleotides or mixture thereof. Examples of the drug include progesterone, haloperidol, thiothixene, olanzapine, clozapine, bromperidol, pimozide, risperidone, ziprasidone, diazepam, ethyl loflazepate, alprazolam, nemonapride, fluoxetine, sertraline, venlafaxine, donepezil, tacrine, galantamine, rivastigmine, selegiline, ropinirole, pergolide, trihexyphenidyl, bromocriptine, benztropine, colchicine, nordazepam, etizolam, bromazepam, clotiazepam, mexazolum, buspirone, goserelin acetate, somatotropin, leuprolide acetate, octreotide, cetrorelix, sandostatin acetate, gonadotropin, fluconazole, itraconazole, mizoribine, cyclosporin, tacrolimus, naloxone, naltrexone, cladribine, chlorambucil, tretinoin, carmustine, anagrelide, doxorubicin, anastrozole, idarubicin, cisplatin, dactinomycin, docetaxel, paclitaxel, raltitrexed, epirubicin, letrozole, mefloquine, promaquine, oxybutynin, tolterodine, allylestrenol, lovastatin, simvastatin, pravastatin, atorvastatin, alendronate, salcatonin, raloxifene, oxadrolone, conjugated estrogen, estradiol, estradiol valerate, estradiol benzoate, ethinyl estradiol, etonogestrel, levonorgestrel, tibolone, and norethisterone, preferably risperidone or progesterone.

Another examples of drug may be interleukin, interferon, tumor necrosis fiactor, insulin, glucagon, growth hormone, gonadotropin, oxytocin, thyroid stimulating hormone, parathyroid hormone, calcitonin, colony stimulation factor, erythropoietin, thrombopoietion, insulin-line growth factor, epidermal growth factor, platelet-derived growth factor, transforming growth factor, fibroblast growth factor, vascular endothelial growth factor or bone morphogenetic protein.

The mixing was performed by conventional method known in the art. For example, using conventional mixer, a drug could be mixed with prepared adhesive composition or a drug could be mixed (or dispersed) with tannin, PEG or water or lower alcohol and deposited into the adhesive composition of the present invention.

The description also discloses use of a composition comprising tannin, poly(ethylene glycol), and water, lower alcohol or a mixture thereof for the manufacture of a anti-adhesion agent.

The description also discloses use of a composition comprising tannin, poly(ethylene glycol), and water, lower alcohol or a mixture thereof for the manufacture of a surface adsorption barrier.

The description also discloses use of a composition comprising tannin, poly(ethylene glycol), and water, lower alcohol or a mixture thereof for the manufacture of a medical hemostatic.

### [Mode for Invention]

The examples and experiments will now be described. The following examples and experiments are for illustrative purposes only and not intended to limit the scope of the present invention.

### <Example 1>

### Preparation of adhesive including tannin, poly(ethylene glycol) and water

Tannin (tannic acid, Chemical Formula 1) is dissolved into 1x phosphate-buffered saline (PBS) buffer (pH 8.5) at a concentration of 1 g/mL. Then, linear or multi-branched poly(ethylene glycol) (PEG) end-capped with -OH, -NH₂ (amine, AM), -SS (succinimidyl succinate) or -SH (thiol) group as shown in Tables 1 and 2 is dissolved into 1x PBS buffer (pH 8.5) at a concentration of 1 g/mL.

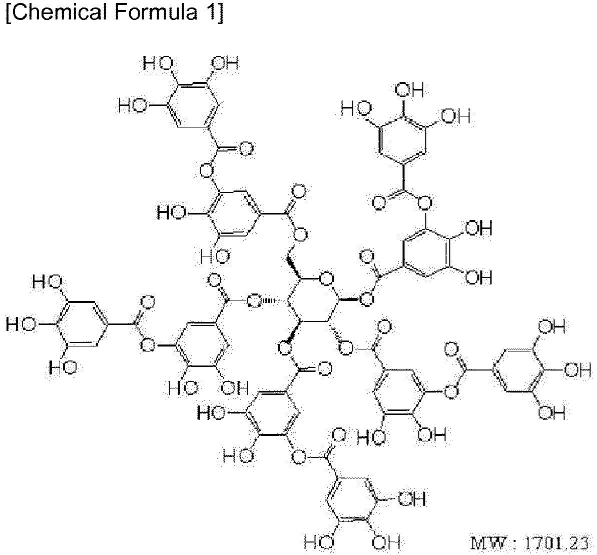

Tannin is mixed with PEG, mPEG-SH, mPEG-AM, mPEG-SS, PEG-AM, 4-arm-PEG-AM, 4-arm-PEG-SS or 6-arm-PEG-AM as shown in Table 1 in such a manner that the molar ratio of pyrogallol: functional group is 25:1. After the resultant mixture is allowed to stand for about 10 minutes, it is separated into a lower layer (brown-colored adhesive) and an upper layer (transparent liquid). Then, the transparent liquid of the upper layer is discarded and the brown-colored adhesive of the lower layer is collected. The brown-colored adhesive thus separated is shown in Fig. 1.

In addition, lower alcohol, ethanol or methanol is used to provide adhesive compositions in the same manner as described above. Tannin (tannic acid, Chemical Formula 1) is dissolved into ethanol or methanol at a concentration of 1 g/mL. Next, 4-arm-PEG-OH is dissolved into ethanol or methanol at a concentration of 1 g/mL, and 4-arm-PEG-AM is dissolved into methanol at a concentration of 1 g/mL. Then, the tannin solution is mixed with the PEG solution at the same ratio as the above-mentioned molar ratio of tannin (dissolved in water) : functional group. The adhesives using lower alcohol are obtained in the same manner as described above.

**[Table 1]**

| | PEG(1 g/mL) | | | Tannin (1g/mL) | Total volume (µL) |
|---|---|---|---|---|---|
| Linear | Type | Molecular weight (Da) | Volume (µL) | Volume (µL) | 200 |
| | PEG | 2k | 38 | 162 | |
| | | 4.6k | 70 | 130 | |
| | mPEG-SH | 5k | 108 | 92 | |
| | mPEG-AM | | | | |
| | mPEG-SS | | | | |
| | PEG-AM | 2k | 38 | 162 | |
| Multi-branched | 4-arm-PEG-AM | 10k | 74 | 126 | |
| | 4-arm-PEG-SS | | | | |
| | 6-arm-PEG-AM | 15k | 74 | 126 | |

### <Example 2>

### Determination of adhesion strength

A biopsy punch (Miltex REF 33-36) is used to cut a pig skin tissue with a diameter of 6 mm. The adhesive according to Example 1 is applied uniformly between two layers of pig skin tissues in an amount of about 3 g, and an instant glue is applied to the back surface so that the pig skin sample is attached to two bars of a system designed to determine adhesion strength. A universal testing machine (INSTRON 5583) is used to apply a force of 20N to the samples for 1 minute and then tensile force is applied thereto at a rate of 1 mm/min to determine the adhesion strength. Adhesives using different types of PEGs are tested continuously at least three times for three samples, and the average adhesion strength and the highest adhesion strength are measured.

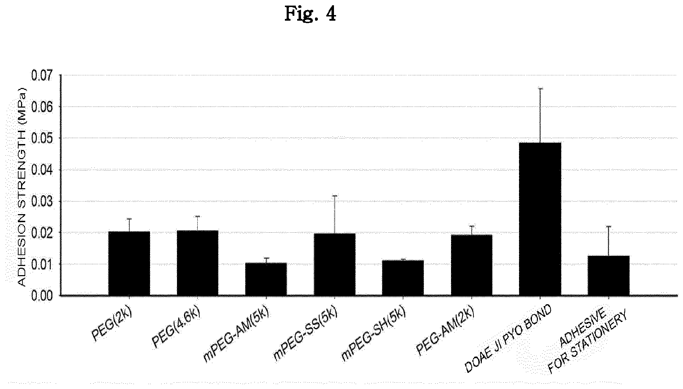

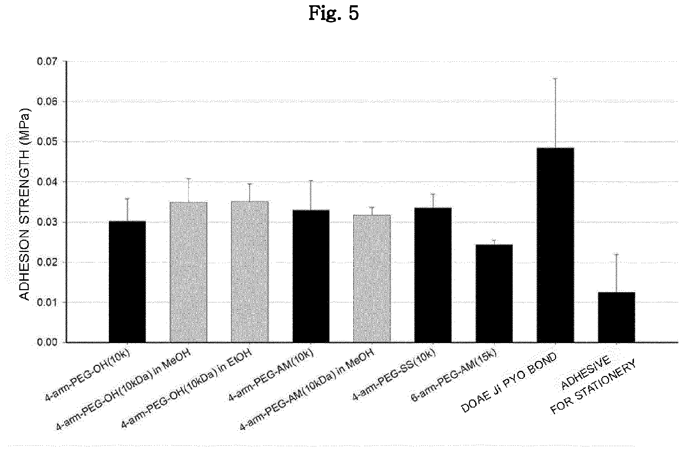

After the test, as can be seen from Fig. 4 and Fig. 5, when using water as a solvent, 4-arm-PEG-AM (10 kDa) and 4-arm-PEG-SS (10 kDa) show the highest adhesion strength of 0.033 MPa, and mPEG-AM (5 kDa) and mPEG-SH (5 kDa) show the lowest adhesion strength of 0.01-0.011 MPa. When using alcohol as a solvent, adhesion strength increases as compared to the adhesives using water as a solvent. Particularly, 4-arm-PEG-OH dissolved in methanol and in ethanol shows the highest adhesion strength of 0.035 MPa, and 4-arm-PEG-AM dissolved in methanol shows a similar adhesion strength of 0.032 MPa.

In addition, the adhesion strength of the adhesive disclosed herein is compared to the adhesion strength of a commercially adhesive for stationery and that of a commercially available bond (Doae Ji Pyo Bond available from Daeheung Chemical Co.)

After the test, as can be seen from Fig. 4 and Fig. 5, mPEG-AM (5 kDa) and mPEG-SH (5 kDa) show a lower adhesion strength than the adhesive for stationery. However, the other adhesives except the two adhesives, i.e., PEG (2kDa, 4.6kDa), mPEG-SS (5kDa), PEG-AM (2kDa), 4-arm-PEG-OH (10kDa), 4-arm-PEG-OH (10kDa) in MeOH, 4-arm-PEG-OH (10kDa) in EtOH, 4-arm-PEG-AM (10kDa), 4-arm-PEG-AM (10kDa) in MeOH, 4-arm-PEG-SS (10kDa), and 6-arm-PEG-AM (15kDa) show a higher adhesion strength than the adhesive for stationery and a lower adhesion strength than the commercially available bond.

### <Example 3>

### Determination of variations in adhesion strength and maintenance time depending on number of repeated adhesion tests

4-arm-PEG-OH (10 kDa) and 4-arm-PEG-AM (10 kDa) adhesives are prepared in the same manner as described in Example 1. In the same manner as described in Example 2, the adhesives are applied uniformly on the pig skin tissues having a diameter of 6 mm in an amount of about 3 g and the pig skin samples are attached to two bars of a system designed to determine adhesion strength by using an instant glue. A universal testing machine (INSTRON 5583) is used to apply a force of 20N to the samples for 1 minute and then tensile force is applied thereto at a rate of 10 mm/min to determine adhesion strength. The above procedure is repeated to determine variations in adhesion strength depending on the number of repeated adhesion tests. One sample of 4-arm-PEG-OH (10 kDa) is tested 82 times continuously for 5 hours, and one sample of 4-arm-PEG-AM (10 kDa) is tested 109 times continuously for 6 hours. Such different numbers of repeated adhesion tests are determined optionally by a test worker due to a limited experimental environment.

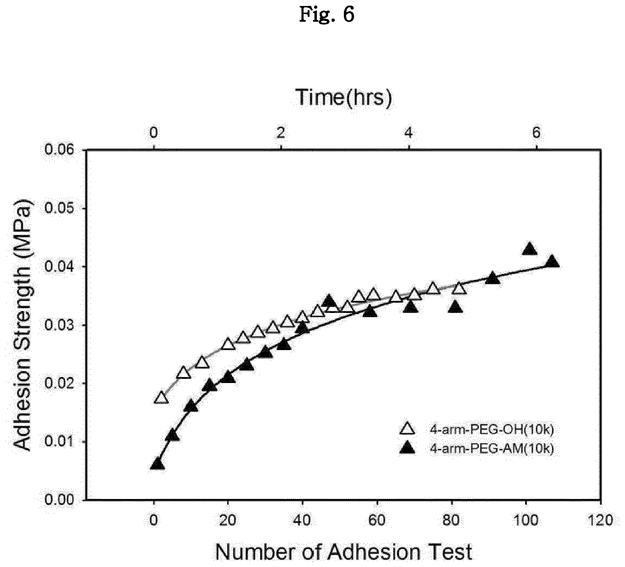

After the test, as can be seen from Fig. 6, both 4-arm-PEG-OH (10 kDa) and 4-arm-PEG-AM (10 kDa) show an increase in adhesion strength as the number of repeated adhesion tests increases. It can be also seen that the adhesives maintain adhesion strength for 5-6 hours after repeating adhesion. This suggests that the adhesive disclosed herein is characterized by its maintenance of adhesion strength after repeating adhesion, unlike conventionally used adhesives that allow adhesion only once or show a decrease in adhesion strength after repeating adhesion. In addition, the adhesion strength of 4-arm-PEG-OH (10 kDa) is compared to that of 4-arm-PEG-AM (10 kDa). After the comparison, it can be seen that although 4-arm-PEG-OH (10 kDa) shows a higher initial adhesion strength than 4-arm-PEG-AM (10 kDa), 4-arm-PEG-AM (10 kDa) shows higher adhesion strength than 4-arm-PEG-OH (10 kDa) at the later part of the tests after repeating adhesion 80-90 times.

### <Example 4>

### Determination of water resistance of adhesive

In the same manner as described in Example 1, 4-arm-PEG-OH (10 kDa) and 4-arm-PEG-AM (10 kDa) adhesives are prepared. In the same manner as described in Example 2, the adhesives are applied uniformly on the pig skin tissues having a diameter of 6 mm in an amount of about 3 g and the pig skin samples are attached to two bars of a system designed to determine adhesion strength by using an instant glue. A universal testing machine (INSTRON 5583) is used to apply a force of 20N to the samples for 1 minute and then tensile force is applied thereto at a rate of 10 mm/min to determine adhesion strength at the first test. Then, the second test ∼ the 11^{th} test are carried out by applying 20 µL of distilled water to the samples each time, and then applying a force of 20N to the samples for 1 minute and applying tensile force thereto at a rate of 10 mm/min under the same condition as in the first test. At the 12^{th} test, a strong dust remover (Nabakem, DR. 747) is used to remove the remaining distilled water, and then a force of 20N is applied to the samples for 1 minute and then tensile force is applied thereto at a rate of 10 mm/min under the same condition as in the previous tests. In this manner, the adhesion strength of each sample is determined until the 21^{st} test is completed.

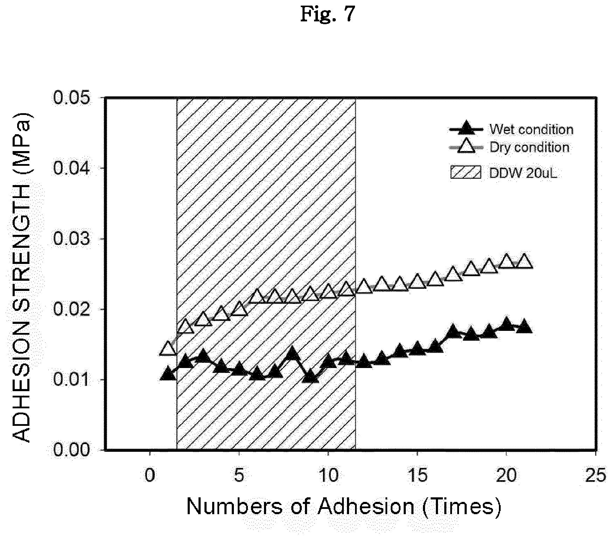

As can be seen from Fig. 7, when the adhesion strength of 4-arm-PEG-OH (10 kDa) is determined after applying 20 µL of distilled water thereto, there is a slight decrease in adhesion strength as compared to the same adhesive to which distilled water is not applied, but the adhesive maintains an adhesion strength of about 0.012 MPa. Then, the adhesion strength is determined continuously after removing distilled water at the 12^{th} test. It can be seen that the adhesion strength increases again after removing distilled water.

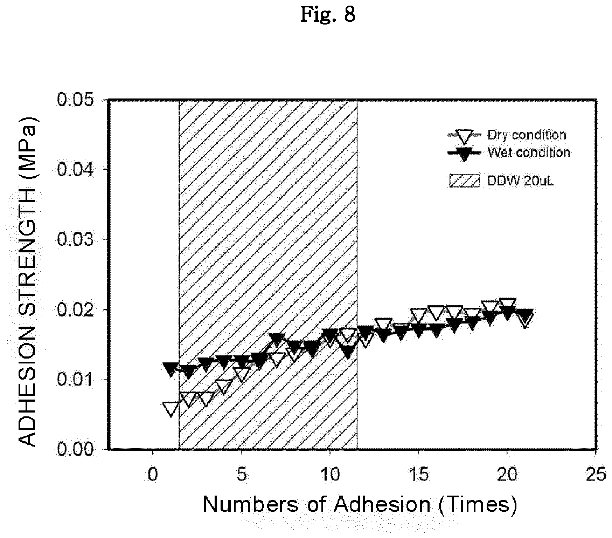

As can be seen from Fig. 8, when the adhesion strength of 4-arm-PEG-AM (10 kDa) is determined after applying 20 µL of distilled water thereto, there is a slight increase in adhesion strength as compared to the same adhesive to which distilled water is not applied. It is thought that such an increase in adhesion strength results from the fact that the adhesion strength determined at the first test is higher than the adhesion strength of the adhesive sample to which distilled water is not applied. The overall graph of the sample to which distilled water is applied shows an increase in adhesion strength despite the application of 20 µL of distilled water. Since the adhesion strength determined after removing distilled water at the 12^{th} test shows no significant increase or change, it can be said that the adhesion strength of 4-arm-PEG-AM (10 kDa) adhesive is not significantly affected by water.

As can be seen from the above results, the adhesive disclosed herein has water resistance unlike other conventional adhesives.

### <Example 5>

### Determination of decomposition time

A gold surface having an area of 1 cm x 1 cm is modified with 4-arm-PEG-AM (10 kDa) adhesive obtained from Example 1 by applying the adhesive uniformly to the surface in an amount of about 10 mg. The surface is introduced into 1 mL of 1x PBS solution (pH 2, pH 7.4 and pH 10) and the amount of tannin released from the surface is measured over time.

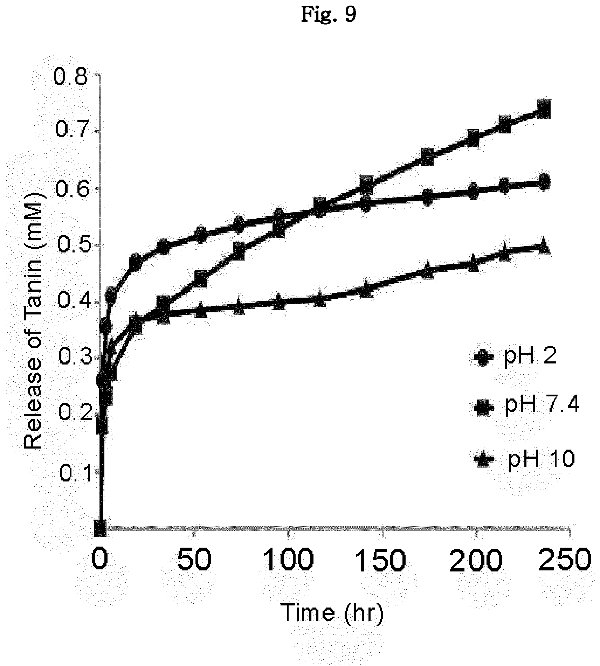

As can be seen from Fig. 9, the adhesive disclosed herein shows different decomposition behaviors depending on pH conditions. At pH 2, the surface is decomposed rapidly only at the initial time, and then is decomposed gradually. At pH 7.4, the surface is decomposed over a long period of time while maintaining stable surface adhesion and the amount of released tannin is in proportion of time. At a high pH of 10, tannin is released from the surface only at the initial time and is hardly released after 20 hours or more. Therefore, when a protein drug for treating diseases is introduced into the adhesive disclosed herein and the drug-containing adhesive is attached to a target surface, it is possible to realize a adhesive depot for sustained release of drug that releases the drug gradually over time as the surface is decomposed.

### <Example 6>

### Determination of drug delivery capabilities

As can be seen from the results of Example 5, incorporation of a protein drug for treating diseases into the adhesive may provide a sustained release formulation that allows sustained release of the drug as the surface is decomposed gradually. Thus, in this Example, bovine serum albumin (BSA) is incorporated into the adhesive of Example 1 in the following ratio to tannin contained in the adhesive: 1/600 (FITC-BSA 50 µL); and 1/300 (FITC-BSA 100 µL). The adhesive is attached to the surface in the same manner as described in Example 5. Then, the amount of protein released from the surface is determined. The amount of protein release is determined by using BSA labeled with a fluorescent material, FITC, and by measuring the emission intensity of the fluorescence upon the excitation with the light having a wavelength of 480 nm.

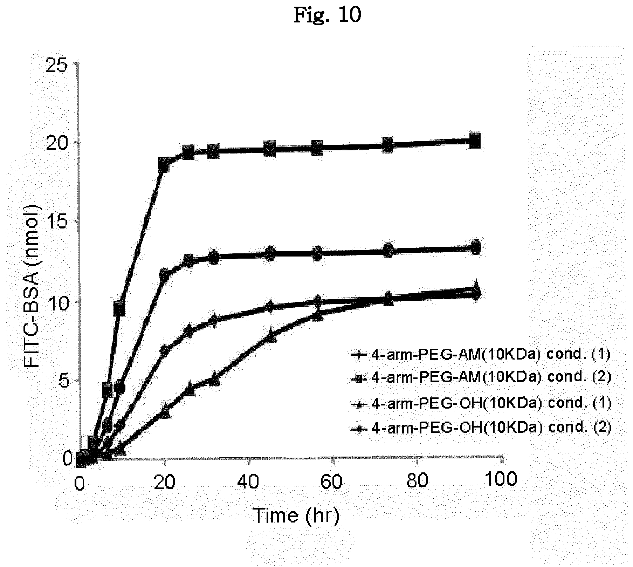

As can be seen from Fig. 10, in the adhesives using 4-arm-PEG-AM (10 kDa) and 4-arm-PEG-OH (10 kDa), incorporation of the protein into the adhesives in an amount of 1/300 of the tannin concentration allows the protein to be released substantially within about 20 hours. Incorporation of the protein into the adhesive using 4-arm-PEG-AM (10 kDa) in an amount of 1/600 of the tannin concentration allows the protein to be released for about 40 hours or more continuously in a concentration that is in proportion to time. Unlike the adhesive using 4-arm-PEG-AM (10 kDa), the adhesive using 4-arm-PEG-OH (10 kDa) under the ratio of protein of 1/600 to tannin allows the protein to be released gradually for an extended period of about 70 hours or more in a concentration that is in proportion to time, wherein the release rate is about 0.15 nmol FITC-BSA/hour. Therefore, it can be seen that from the above results that the drug formulation using the adhesive disclosed herein allows protein release in proportion to time, and thus may be used as a drug delivery system that releases a drug in vivo at a constant rate.

### <Example 7>

### Determination of adhesion strength of adhesive having drug delivery capability

In the test of Example 6, the adhesives are evaluated for their drug delivery capabilities. In this Example, effects of the incorporation of protein into the adhesives upon the adhesion strength of the adhesives are determined. First, the reagents are mixed in the amounts as shown in Table 2. The reagents are mixed in the manner as described hereinafter. To 30 µL of 4-arm-PEG solution, different amounts of FITC-BSA (albumin-fluorescein isothiocyanate conjugate, Sigma) solutions are mixed thoroughly, and then 1x PBS buffer (pH 7.4) is used to adjust the total volumes of the resultant solutions to predetermined volumes. To the resultant solutions, 50 µL of tannin solution is added, followed by mixing, to provide adhesives. The adhesives thus obtained are determined for their adhesion strengths in the same manner as described in Example 2. However, the universal testing machine is used at a speed of 10 mm/min and the adhesion test is carried out 15 times for each sample.

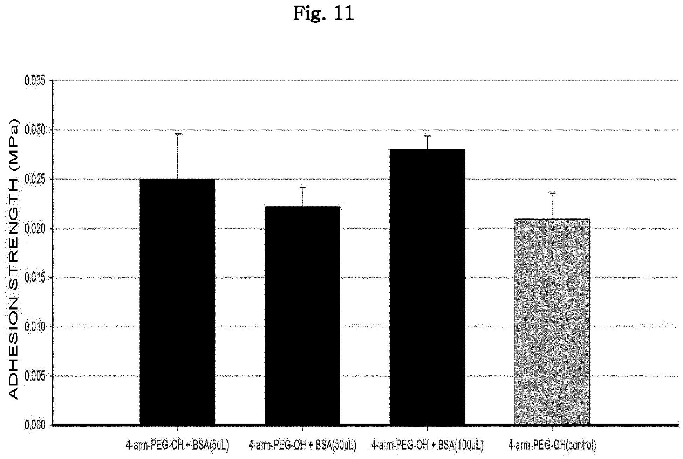

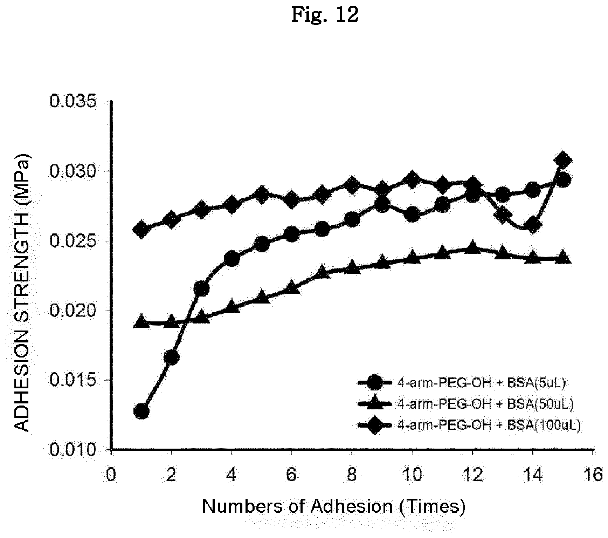

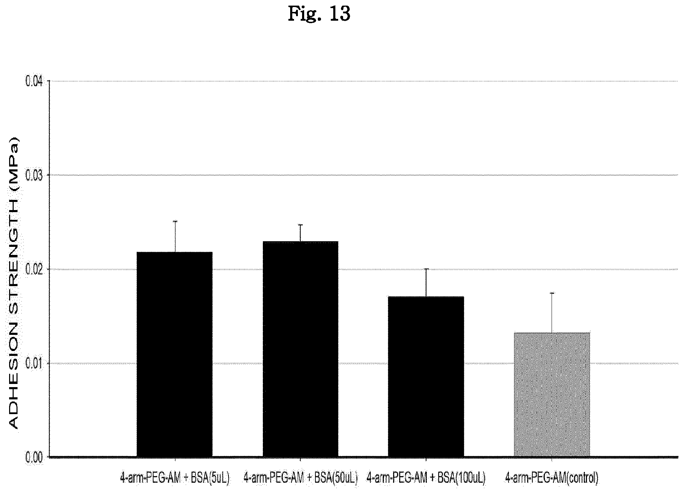

As can be seen from Fig. 11 and Fig. 12, it is shown that the amount of FITC-BSA has no effect on the adhesion strength of 4-arm-PEG-OH (10 kDa). However, the adhesive containing FITC-BSA has higher adhesion strength than the original adhesive. As can be seen from Fig. 13 and Fig. 14, 4-arm-PEG-AM (10 kDa) containing FITC-BSA also has higher adhesion strength than the original adhesive in a similar manner to 4-arm-PEG-OH. Also, it can be seen that when formulating a practical adhesive containing FITC-BSA, the viscosity of the adhesive increases in proportion to the amount of FITC-BSA, and the adhesive becomes a gum-like state.

**[Table 2]**

| | 4-arm-PEG-OH(1g/mL) | 4-arm-PEG-AM(1g/mL) | FITC-BSA (1mM) | 1xPBS (pH 7.4) | Tannic acid (1g/mL) |
|---|---|---|---|---|---|
| 1 | 30 µL | 0 µL | 5 µL | 95 µL | 50 µL |
| 2 | 30 µL | 0 µL | 50 µL | 50 µL | 50 µL |
| 3 | 30 µL | 0 µL | 100 µL | 0 µL | 50 µL |
| 4 | 0 µL | 30 µL | 5 µL | 95 µL | 50 µL |
| 5 | 0 µL | 30 µL | 50 µL | 50 µL | 50 µL |
| 6 | 0 µL | 30 µL | 100 µL | 0 µL | 50 µL |

### <Example 8>

### Determination of capability as anti-adhesion agent against cells and proteins on surfaces

A gold surface is modified by applying the adhesive obtained from Example 1 uniformly to an area of 1 cm x 1 cm in an amount of about 10 mg in the same manner as described in Example 5. Two types of PEGs, 4-arm-PEG-AM (10 kDa) and 4-arm-PEG-OH (10 kDa) are used to provide the following adhesives:
Adhesive prepared at room temperature (Condition 1),
Adhesive prepared by warming PEG to 60°C and adding tannin thereto (Condition 2), and
Adhesive prepared by mixing PEG with tannin at room temperature and storing the mixture at 60°C for 1 hour (Condition 3).

The gold surface modified with each type of adhesive is introduced into a cell culture (DMEM containing 10% FBS) for 1 hour and then removed. Then, the gold surface is further introduced into a cell culture containing NIH3T3 cells to culture them for 12 hours. The nuclei of the cells attached to the surface are subjected to fluorescence staining (DAPI) and observed with a fluorescence microscope. In addition, the above three types of adhesives are determined for their capabilities as anti-adhesion agents.

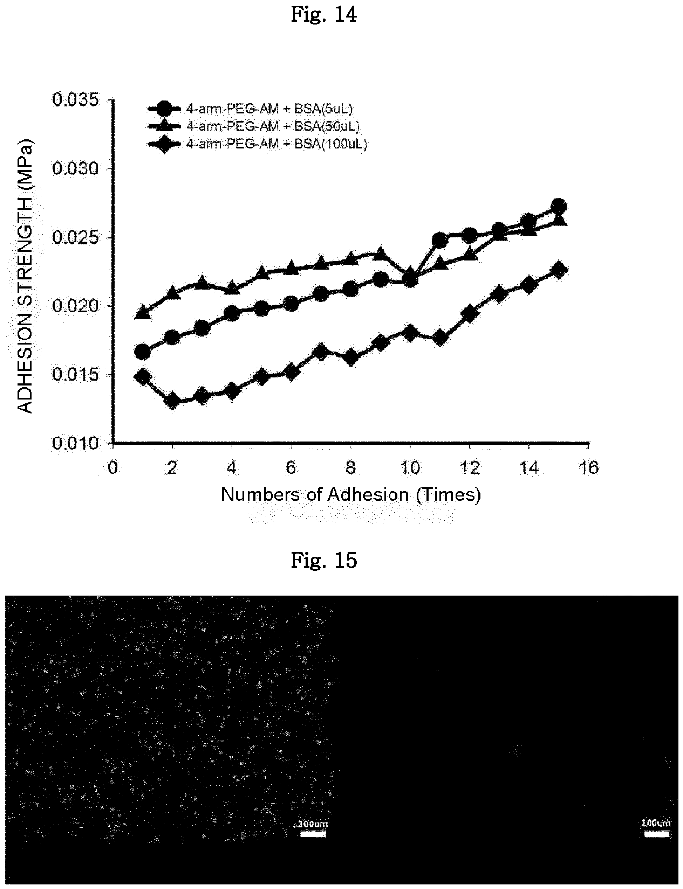

After the test, as can be seen from Fig. 15, it is shown that the cells are still attached to the non-modified surface, while they remain on the modified surface in a significantly reduced number as compared to the non-modified surface.

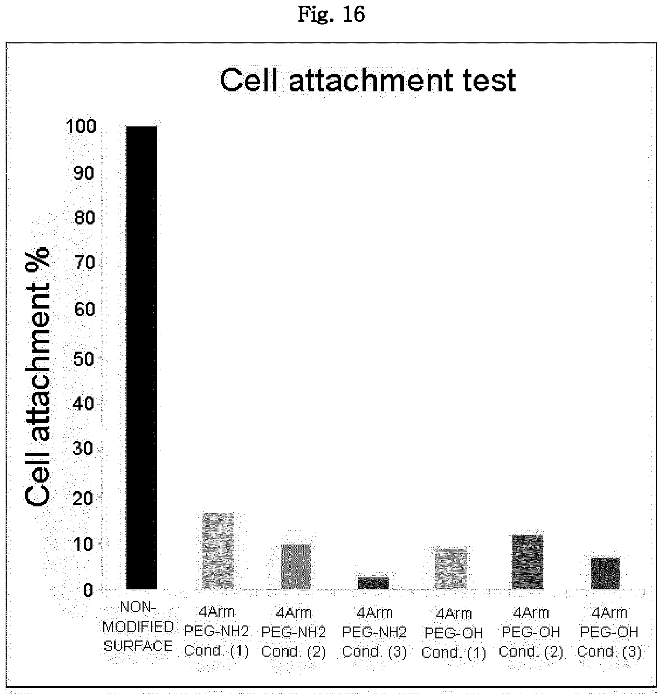

More particularly, as can be seen from Fig. 16, the surface modified with the adhesive using 4-arm-PEG-AM (10 kDa) shows a significantly lower ratio of cell attachment. As compared to the non-modified surface, Condition 1 provides 16.5% of cell attachment and Condition 2 provides 9.78% of cell attachment. Particularly, the surface modified with the adhesive using 4-arm-PEG-AM (10 kDa) and stored at 60°C for 1 hour (Condition 3) provides the highest capability as an anti-adhesion agent as evidenced by the cell attachment ratio of 2.57% as compared to the non-modified surface. In the case of 4-arm-PEG-OH (10 kDa), Condition 1 provides 8.68% of cell attachment, Condition 2 provides 11.99% of cell attachment, and Condition 3 provides 6.84% of cell attachment, as compared to the non-modified surface. Thus, it can be seen that 4-arm-PEG-OH (10 kDa) does not show a significant difference in its capability as an anti-adhesion agent under different conditions of temperature or procedure.

### <Example 9>

### Determination of decomposition time

To determine the natural degradability of the adhesive composition disclosed herein, the degradability of the adhesive is evaluated by measuring the weight of the adhesive composition when the adhesive is placed at a temperature similar to the human body temperature in an excessive amount of aqueous solution.

For this, PEG (4.6k) adhesive compositions are obtained in the same manner as described in Example 1 so that the molar ratio of pyrogallol : functional group is 10:1 or 30:1.

To determine the decomposition time, a cylindrical shaped container is obtained by cutting the lid of a microtube (1.5 mL). Then, the container is attached to a Petri dish by using a strong adhesive, thereby providing a system for measuring the weight of an adhesive. First, the weight of the resultant system is measured. Next, the preliminarily formed PEG (4.6k) 10:1 and 30:1 adhesives each are introduced into a microtube container in an amount of 50 mg. Then, 35 mL of 1x PBS buffer (pH 7.4) is added thereto so that the microtube container having the PEG (4.6k) adhesive is immersed completely. The system is placed in an incubator and allowed to stand at 37°C under 50 rpm for a predetermined time. After that, the system is removed from the incubator and 1x PBS buffer is discarded. Then, the system is dehydrated completely, for example, by nitrogen blowing, and the weight is measured.

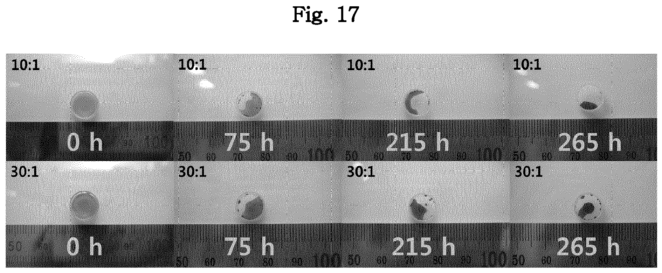

After the test, as can be seen from Fig. 17, both adhesives having a molar ratio of 10:1 and 30:1 are decomposed naturally with time. A decrease in amount of the adhesives may be observed with the naked eye. In addition, it can be seen that the adhesives initially having a yellow color turn into a brown color with time. After measuring the weight, the weight increases at the initial time (it is thought that such an increase results from water absorption), but the PEG (4.6k) 10:1 adhesive and the PEG (4.6k) 30:1 adhesive show a decrease in weight after 4 hours and 11 hours, respectively.

Therefore, it is believed that although the adhesive disclosed herein has water resistance, it may be decomposed after retained in the living body for a long time.

### <Example 10>

### Determination of effect of adhesive upon hemostasis

The following test for determining the effect of the adhesive upon hemostasis is based on Y. Murakami et al., Colloids and Surfaces B: Biointerfaces 65 (2008) 186-189. Particularly, the test is carried out in the manner described hereinafter.

An ICR mouse (7-8 week-aged, male) is used. To measure the amount of blood, filter paper on which blood is to be absorbed is cut into a predetermined size and weighed in advance. A parafilm, which is used to prevent intestinal juices or blood from infiltrating into the filter paper, is cut into a size slightly larger than the filter paper. To measure the amount of blood for 2 minutes at an interval of 30 seconds, the preliminarily weighed filter paper and parafilm are stacked four-fold in the order of filter paper-parafilm-filter paper-parafilm. After anesthetizing the mouse, it is fixed to a styrofoam plate and its belly is cut. The styrofoam plate to which the mouse is fixed is tilted at 45°, and the intestinal juices present at the liver of the mouse are wiped off. Then, the stack of filter paper with parafilm is placed under the liver of the mouse, and the center of the liver is pierced obliquely with a 19G syringe needle. While the filter paper is exchanged at an interval of 30 seconds, blood flowing out for 2 minutes is absorbed on the filter paper to measure the amount of blood. In this manner, the amount of blood is determined.

In the case of the test group, a PEG (4.6k) adhesive is obtained in the same manner as described in Example 1 so that the ratio of pyrogallol:functional group = 30:1. After the liver of the mouse is pierced with a 19G syringe needle, 0.1 mL of the adhesive is applied thereto. In the case of a negative control, the liver is non-treated. Meanwhile, a positive control is prepared by applying Fibrin glue (Beriplast P Combi set, CSL Behring, Germany) to the liver. Herein, Fibrin glue flows down together with blood. Thus, Fibrin glue is also applied to a liver without any cut, and the weight of Fibrin glue absorbed on filter paper is measured so that it is subtracted from the measurement of the positive control.

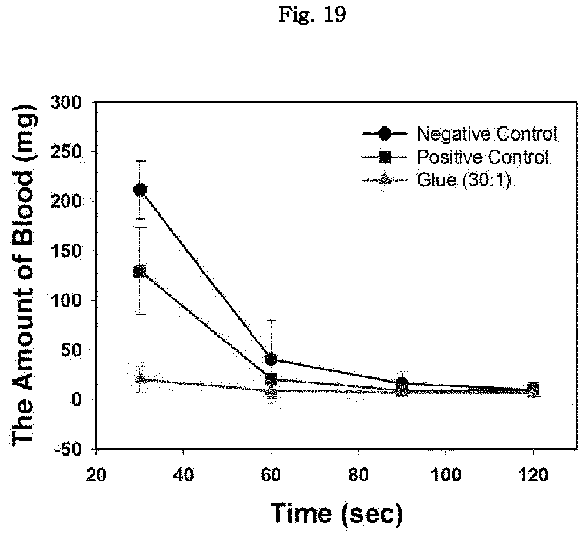

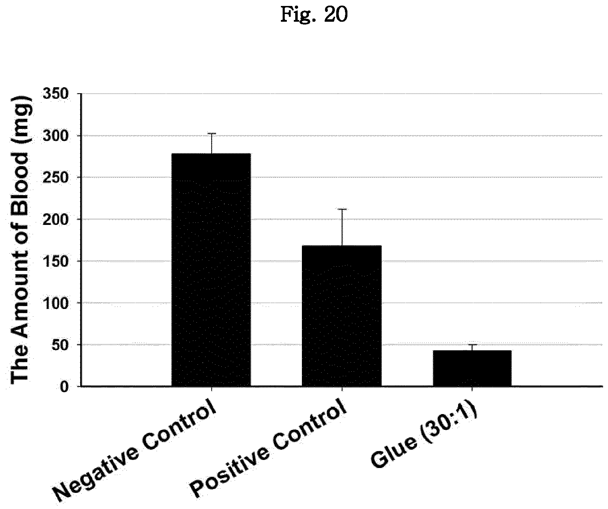

After the test, as can be seen from Fig. 19 and Fig. 20, the PEG (4.6k) adhesive disclosed herein shows an excellent hemostatic effect.

Referring to Fig. 19, the amount of blood during the initial 30 seconds is about 20 mg in the case of the PEG (4.6k) adhesive disclosed herein. This is significantly lower than the negative control (210 mg). The amount of blood of Fibrin glue as a positive control is about 130 mg. Therefore, it can be seen that the PEG (4.6k) adhesive disclosed herein shows a better hemostatic effect than the commercialized hemostatic agent, Fibrin glue.

Referring to Fig. 20 showing the total amount of blood flowing out for 2 minutes, the total amount of blood of the non-treated negative control is 277 mg, that of Fibrin glue as a positive control is 168 mg, and that of the PEG (4.6k) adhesive disclosed herein is 42 mg. In brief, the PEG (4.6k) adhesive disclosed herein shows a total amount of blood corresponding to 1/7 of the negative control and 1/4 of Fibrin glue. This demonstrates that the adhesive disclosed herein has an excellent hemostatic effect.

### <Example 11>

### Skin sensitivity test of adhesive

The skin sensitivity test of the adhesive disclosed herein is made by a service of Korea Testing & Research Institute.

To evaluate the skin sensitivity of the adhesive, a Hartley guinea pig is subjected to a test according to ISO 10993-10:2002/Amd.1:2006(E), maximization test for delayed hypersensitivity. Eluate obtained by eluting the adhesive with sterilized saline and cotton seed oil in an amount of 20 mL per 4 g of adhesive at a temperature of 37°C for 72 hours is injected via intradermal injection to induce first sensitization, and then is applied transdermally to induce second sensitization. The above procedure is repeated merely by using sterilized saline and cotton seed oil, and the thus treated samples are used as negative controls. The sensitized skin site is evaluated 24 hours and 48 hours after the completion of the exposure to the eluate for challenging sensitization. The test groups and administration doses are shown in the following Table 3.

**[Table 3]**

| Group | Test materials | |
|---|---|---|
| | Induction | Challenge |
| Test group (sterilized saline) | Sterilized saline eluate of adhesive 0.1 mL | Sterilized saline eluate of adhesive 0.1 mL |
| Negative control (sterilized saline) | Blank (sterilized saline) 0.1 mL | Sterilized saline eluate of adhesive 0.1 mL |
| Test group (cotton seed oil) | Cotton seed oil eluate of adhesive 1 mL | Cotton seed oil eluate of adhesive 1 mL |
| Negative control (cotton seed oil) | Blank (cotton seed oil) 0.1 mL | Cotton seed oil eluate of adhesive 1 mL |

All the treated animals show no clinical conditions, or no animals are on the verge of death or fall dead. After measuring the body weights, both the test group and the negative control show a normal increase in body weight. Over the whole period of test, the skin sites treated with the test material show no irritation conditions, including erythema, callus and edema.

As can be seen from the foregoing results, the adhesive disclosed herein has little skin sensitivity to Hartley guinea pigs. It is believed that such results are derived from the fact that the composition disclosed herein includes tannin, PEG and water or lower alcohol.

### <Example 12>

### Skin irritation test of adhesive

The skin irritation test of the adhesive disclosed herein is made by a service of Korea Testing & Research Institute.

To evaluate the skin irritability of the adhesive, New Zealand White male rabbits are subjected to a test according to ISO 10993-10 animal skin irritation test. Eluate obtained by eluting the adhesive with sterilized saline and cotton seed oil in an amount of 20 mL per 4 g of adhesive at a temperature of 37°C for 72 hours is applied to skin samples with a size of 2.5 cm X 2.5 cm in an amount of 0.5 mL for 4 hours. Then, mortality, general conditions, weight changes and local irritability are evaluated for 72 hours.

After observing general conditions during the test, there are no specific clinical conditions and dead animals. After measuring the body weight of each animal subject upon the introduction of animals, right before the application of the eluate, and 24 hours and 72 hours after the application of the eluate, a normal increase in body weight is observed. After observing the skin samples 1 hour, 24 hours, 48 hours and 72 hours after the application of the test material, there are no skin responses, including erythema, callus and edema. Thus, the primary irritation index (PII) is evaluated as "0.0".

As can be seen from the foregoing results, application of the adhesive disclosed herein causes no erythema, callus and edema in New Zealand White male rabbits. Finally, the skin irritability of the test material is regarded as 'negligible'.

### [Industrial Applicability]

The adhesive composition disclosed herein includes tannin, poly(ethylene glycol) and water. The adhesive composition has little toxicity, allows adhesion even in the absence of a thermosetting curing agent, and is hardly soluble in water to show moisture resistance, unlike known tannin adhesives. Therefore, the adhesive composition may be applied as a medical adhesive, a adhesive depot for sustained release of drug, a anti-adhesion agent, a cell/protein adsorption barrier and a medical hemostatic.

## Claims

1. An adhesive composition for use in a method of adhering tissue or organs in vivo, the adhesive composition comprising: tannin; poly(ethylene glycol); and a C1-C6 alcohol, wherein the poly(ethylene glycol) has a molecular weight of 1,000-100,000 Dalton.

2. The adhesive composition according to claim 1, wherein the tannin has a molecular weight of 500-10,000 Dalton.

3. The adhesive composition according to claim 1, wherein the poly(ethylene glycol) comprises a functional group selected from the group consisting of: hydroxyl (-OH), amine (-NH₂), succinimidyl succinate, succinic acid, thiol (-SH), acrylate, epoxide, maleimide, nitrophenyl carbonate, orthopyridyl disulfide, tosylate, azide, phosphate, oligoamine ([-CH₂-CH₂-NH-]ₙ), catechol and catecholamine.

4. A medical adhesive comprising the adhesive composition as defined in claim 1.

5. An adhesive depot for sustained release of a drug or mixture of drugs comprising the adhesive composition as defined in claim 1.

6. The adhesive depot according to claim 5, which comprises a hardly soluble drug, therapeutic peptide, protein or antibody.

7. An anti-adhesive agent comprising the adhesive composition as defined in claim 1.

8. A surface adsorption barrier comprising the adhesive composition as defined in claim 1.

9. A medical hemostatic comprising the adhesive composition as defined in claim 1.

10. A method for preparing an adhesive composition for use in adhering tissue or organs in vivo, comprising:
(a) mixing tannin, poly(ethylene glycol), and a C1-C6 alcohol, wherein the poly(ethylene glycol) has a molecular weight of 1,000-100,000 Dalton; and
(b) acquiring the resultant mixture as an adhesive composition.

11. A method for preparing an adhesive composition for use in adhering tissue or organs in vivo, comprising:
(a) dissolving poly(ethylene glycol) in a C1-C6 alcohol, wherein the poly(ethylene glycol) has a molecular weight of 1,000-100,000 Dalton;
(b) dissolving tannin in a C1-C6 alcohol;
(c) mixing the solutions obtained from step (a) and step (b) with each other to form an adhesive composition; and
(d) acquiring the resultant adhesive composition.

12. An adhesive composition obtained by the method as defined in claim 10 or 11.

## Patentansprüche

1. Klebstoffzusammensetzung zur Verwendung in einem Verfahren zum Verkleben von Gewebe oder Organen in vivo, wobei die Klebstoffzusammensetzung Tannin; Poly(ethylenglykol); und einen C1-C6-Alkohol aufweist, wobei das Poly(ethylenglykol) ein Molekulargewicht von 1.000-100.000 Dalton hat.

2. Klebstoffzusammensetzung nach Anspruch 1, wobei das Tannin ein Molekulargewicht von 500-10.000 Dalton hat.

3. Klebstoffzusammensetzung nach Anspruch 1, wobei das Poly(ethylenglykol) eine funktionelle Gruppe aufweist, ausgewählt aus der Gruppe bestehend aus: Hydroxyl (-OH), Amin (-NH₂), Succinimidylsuccinat, Bernsteinsäure, Thiol (-SH), Acrylat, Epoxid, Maleimid, Nitrophenylcarbonat, Orthopyridyldisulfid, Tosylat, Azid, Phosphat, Oligoamin ([-CH₂-CH₂-NH-]ₙ), Katechol und Katecholamin.

4. Medizinischer Klebstoff, der die Klebstoffzusammensetzung nach Anspruch 1 aufweist.

5. Klebstoffdepot zur verzögerten Freisetzung eines Arzneimittels oder einer Mischung von Arzneimitteln, das die Klebstoffzusammensetzung nach Anspruch 1 aufweist.

6. Klebstoffdepot nach Anspruch 5, das ein schwerlösliches Arzneimittel, therapeutisches Peptid, Protein oder Antikörper aufweist.

7. Antihaftmittel, das die Klebstoffzusammensetzung nach Anspruch 1 aufweist.

8. Oberflächenadsorptionsbarriere, die die Klebstoffzusammensetzung nach Anspruch 1 aufweist.

9. Medizinisches Hämostatikum, das die Klebstoffzusammensetzung nach Anspruch 1 aufweist.

10. Verfahren zur Herstellung einer Klebstoffzusammensetzung zur Verwendung zum Verkleben von Gewebe oder Organen in vivo, umfassend:
(a) Mischen von Tannin, Poly(ethylenglykol) und einem C1-C6-Alkohol, wobei das Poly(ethylenglykol) ein Molekulargewicht von 1.000 bis 100.000 Dalton hat; und
(b) Erhalten der resultierenden Mischung als Klebstoffzusammensetzung.

11. Verfahren zur Herstellung einer Klebstoffzusammensetzung zur Verwendung zum Verkleben von Gewebe oder Organen in vivo, umfassend:
(a) Lösen von Poly(ethylenglykol) in einem C1-C6-Alkohol, wobei das Poly(ethylenglykol) ein Molekulargewicht von 1.000 bis 100.000 Dalton hat;
(b) Lösen von Tannin in einem C1-C6-Alkohol;
(c) Mischen der aus Schritt (a) und Schritt (b) erhaltenen Lösungen miteinander, um eine Klebstoffzusammensetzung zu bilden; und
(d) Erhalten der resultierenden Klebstoffzusammensetzung.

12. Klebstoffzusammensetzung, erhalten durch das Verfahren nach Anspruch 10 oder 11.

## Revendications

1. Composition adhésive pour une utilisation dans un procédé d'adhésion de tissu ou d'organes *in vivo,* la composition adhésive comprenant : du tanin ; du polyéthylèneglycol ; et un alcool en C₁ à C₆, où le polyéthylène glycol a un poids moléculaire allant de 1000 à 100000 Dalton.

2. Composition adhésive selon la revendication 1, dans laquelle le tanin a un poids moléculaire allant de 500 à 10000 Dalton.

3. Composition adhésive selon la revendication 1, dans laquelle le polyéthylèneglycol comprend un groupe fonctionnel choisi dans le groupe constitué par : un hydroxyle (-OH), une amine (-NH₂), le succinate de succinimidyle, l'acide succinique, un thiol (-SH), un acrylate, un époxyde, un maléimide, le carbonate de nitrophényle, le disulfure d'orthopyridyle, un tosylate, un azoture, un phosphate, une oligoamine ([-CH₂-CH₂-NH-]ₙ), le catéchol et une catécholamine.

4. Adhésif médical comprenant la composition adhésive telle que définie dans la revendication 1.

5. Dépôt adhésif pour libération prolongée d'un médicament ou d'un mélange de médicaments comprenant la composition adhésive telle que définie dans la revendication 1.

6. Dépôt adhésif selon la revendication 5, qui comprend un médicament difficilement soluble, un peptide thérapeutique, une protéine ou un anticorps.

7. Agent antiadhésif comprenant la composition adhésive telle que définie dans la revendication 1.

8. Barrière d'adsorption superficielle comprenant la composition adhésive telle que définie dans la revendication 1.

9. Hémostatique médical comprenant la composition adhésive telle que définie dans la revendication 1.

10. Procédé de préparation d'une composition adhésive pour une utilisation dans l'adhésion de tissu ou d'organes *in vivo,* comprenant le fait :
(a) de mélanger le tanin, le polyéthylèneglycol et un alcool en C₁ à C₆, où le polyéthylèneglycol a un poids moléculaire allant de 1000 à 100000 Dalton ; et
(b) d'acquérir le mélange résultant en tant que composition adhésive.

11. Procédé de préparation d'une composition adhésive pour une utilisation dans l'adhésion de tissu ou d'organes *in vivo,* comprenant le fait :
(a) de dissoudre le polyéthylèneglycol dans un alcool en C₁ à C₆, où le polyéthylèneglycol a un poids moléculaire allant de 1000 à 100000 Dalton ;
(b) de dissoudre le tanin dans un alcool en C₁ à C₆ ;
(c) de mélanger les solutions obtenues à l'étape (a) et à l'étape (b) l'une avec l'autre pour former une composition adhésive ; et
(d) d'acquérir la composition adhésive résultante.

12. Composition adhésive obtenue par le procédé tel que défini dans la revendication 10 ou 11.
